(19) Europäisches Patentamt
European Patent Office
Office européen des brevets

(11) **EP 3 759 233 B1**

(12) **EUROPEAN PATENT SPECIFICATION**

(45) Date of publication and mention
of the grant of the patent:
**30.03.2022 Bulletin 2022/13**

(51) International Patent Classification (IPC):
***C12Q 1/66*** *(2006.01)*  ***G01N 33/542*** *(2006.01)*
***G01N 33/58*** *(2006.01)*  ***G01N 33/68*** *(2006.01)*

(21) Application number: **19721157.6**

(22) Date of filing: **26.02.2019**

(52) Cooperative Patent Classification (CPC):
**G01N 33/542; C12Q 1/66; G01N 33/582;
G01N 33/6845**

(86) International application number:
**PCT/NL2019/050122**

(87) International publication number:
**WO 2019/164402 (29.08.2019 Gazette 2019/35)**

(54) **BIOLUMINESCENT BIOSENSOR FOR DETECTING AND QUANTIFYING BIOMOLECULES**

BIOLUMINESZENTER BIOSENSOR ZUM NACHWEIS UND ZUR QUANTIFIZIERUNG VON BIOMOLEKÜLEN

BIOCAPTEUR BIOLUMINESCENT PERMETTANT DE DETECTER ET DE QUANTIFIER DES BIOMOLECULES

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **26.02.2018 US 201862635053 P**

(43) Date of publication of application:
**06.01.2021 Bulletin 2021/01**

(73) Proprietor: **Technische Universiteit Eindhoven
5612 AE Eindhoven (NL)**

(72) Inventors:
• **MERKX, Maarten**
  **5612 AZ Eindhoven (NL)**
• **NI, Yan**
  **5612 AZ Eindhoven (NL)**
• **VAN AALEN, Eva Anna**
  **5612 AZ Eindhoven (NL)**

(74) Representative: **Algemeen Octrooi- en
Merkenbureau B.V.
P.O. Box 645
5600 AP Eindhoven (NL)**

(56) References cited:
**WO-A1-2015/067302**

• **SAMBASHIVA BANALA ET AL: "No washing, less
waiting: engineering biomolecular reporters for
single-step antibody detection in solution",
ORGANIC & BIOMOLECULAR CHEMISTRY, vol.
11, no. 44, 1 January 2013 (2013-01-01), page
7642, XP55592786, ISSN: 1477-0520, DOI:
10.1039/c3ob41315b**
• **RALPH P. G. BOSMANS ET AL: "Supramolecular
Control over Split-Luciferase Complementation",
ANGEWANDTE CHEMIE, INTERNATIONAL
EDITION, vol. 55, no. 31, 29 June 2016
(2016-06-29) , pages 8899-8903, XP055592785, DE
ISSN: 1433-7851, DOI: 10.1002/anie.201602807**
• **REMCO ARTS ET AL: "Semisynthetic
Bioluminescent Sensor Proteins for Direct
Detection of Antibodies and Small Molecules in
Solution", ACS SENSORS, vol. 2, no. 11, 2
November 2017 (2017-11-02), pages 1730-1736,
XP55592784, ISSN: 2379-3694, DOI:
10.1021/acssensors.7b00695**
• **MARTIJN VAN ROSMALEN ET AL: "Dual-Color
Bioluminescent Sensor Proteins for Therapeutic
Drug Monitoring of Antitumor Antibodies",
ANALYTICAL CHEMISTRY, vol. 90, no. 5, 14
February 2018 (2018-02-14), pages 3592-3599,
XP055592787, US ISSN: 0003-2700, DOI:
10.1021/acs.analchem.8b00041**

SEQUENCE LISTING

<110> Technische Universiteit Eindhoven

<120> Bioluminescent biosensor for detecting and quantifying biomolecules or ligands in solution

<130> 77659PC

<150> US62/635053
<151> 2018-02-26

<160> 27

<170> BiSSAP 1.3.6

<210> 1
<211> 11
<212> PRT
<213> Oplophorus gracilirostris

<220>
<223> SBiT with K(d) of 190 μM

<400> 1

```
Val Thr Gly Tyr Arg Leu Phe Glu Glu Ile Leu
1               5                   10
```

<210> 2
<211> 11
<212> PRT
<213> Oplophorus gracilirostris

<220>
<223> SBiT with K(d) of 2.5 μM

<400> 2

```
Val Thr Gly Tyr Arg Leu Phe Glu Lys Glu Ser
1               5                   10
```

<210> 3
<211> 11
<212> PRT
<213> Oplophorus gracilirostris

<220>
<223> SBiT with K(d) of 0.18 μM

<400> 3

```
Val Thr Gly Tyr Arg Leu Phe Glu Lys Ile Ser
1               5                   10
```

<210> 4
<211> 12
<212> PRT
<213> Artificial Sequence

<220>
<223> HIV1-p17-antibody binding epitope

<400> 4

```
Glu Leu Asp Arg Trp Glu Lys Ile Arg Leu Arg Pro
1               5                   10
```

<210> 5
<211> 12
<212> PRT
<213> Artificial Sequence

<220>
<223> Trastuzumab binding mimitope

<400> 5

```
Gln Leu Gly Pro Tyr Glu Leu Trp Glu Leu Ser His
1               5                   10
```

<210> 6
<211> 407
<212> PRT
<213> Artificial Sequence

<220>
<223> NB-LUMABS-1

<400> 6

```
Glu Leu Asp Arg Trp Glu Lys Ile Arg Leu Arg Pro
1               5                   10
```

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
    50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
            275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Glu Leu Asp Arg Trp Glu Lys
        355                 360                 365
Ile Arg Leu Arg Pro Thr Ser Gly Gly Ser Cys Gly Gly Ser Val Thr
    370                 375                 380
Gly Tyr Arg Leu Phe Glu Lys Glu Ser Gly Gly Ser Gly Gly Ser Trp
385                 390                 395                 400
Ser His Pro Gln Phe Glu Lys
            405
```

<210> 7
<211> 407
<212> PRT
<213> Artificial Sequence

<220>
<223> NB-LUMABS-2

<400> 7

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
        195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
                245                 250                 255
Gly Gly Ser Gly Ala Glu Ala Ala Ala Lys Glu Ala Ala Ala Lys Glu
            260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
    290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
                325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Glu Leu Asp Arg Trp Glu Lys
        355                 360                 365
Ile Arg Leu Arg Pro Thr Ser Gly Gly Ser Gly Gly Ser Val Thr Gly
    370                 375                 380
Tyr Arg Leu Phe Glu Lys Glu Ser Gly Gly Ser Cys Gly Gly Ser Trp
385                 390                 395                 400
Ser His Pro Gln Phe Glu Lys
                405
```

<210> 8

<211> 408

<212> PRT

<213> Artificial Sequence

<220>
<223> NB-LUMABS-3

<400> 8

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
            115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
            260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
    290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Glu Leu Asp Arg Trp Glu Lys
        355                 360                 365
Ile Arg Leu Arg Pro Thr Ser Gly Gly Ser Cys Gly Gly Ser Val Thr
370                 375                 380
Gly Tyr Arg Leu Phe Glu Lys Glu Ser Gly Gly Ser Cys Gly Gly Ser
385                 390                 395                 400
Trp Ser His Pro Gln Phe Glu Lys
                405
```

<210> 9
<211> 407

<212> PRT
<213> Artificial Sequence

<220>
<223> NB-LUMABS-4

<400> 9

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
            35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
            275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
                325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Glu Leu Asp Arg Trp Glu Lys
            355                 360                 365
Ile Arg Leu Arg Pro Thr Ser Gly Gly Ser Cys Gly Gly Ser Val Thr
            370                 375                 380
Gly Tyr Arg Leu Phe Glu Lys Ile Ser Gly Gly Ser Gly Gly Ser Trp
385                 390                 395                 400
Ser His Pro Gln Phe Glu Lys
                405
```

<210> 10
<211> 407
<212> PRT
<213> Artificial Sequence

<220>
<223> NB-LUMABS-5

<400> 10

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
                20                  25                  30
Gly Ser Gly Gly Ser Gly Gly Ser Gly Gly Ser Gly Gly Ser Gly Gly
            35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
                100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
```

```
                275                      280                        285
        Ala Ala Lys Ala Gly Ser Gly Gly Ser Gly Gly Ser Gly Gly Ser Gly
            290                      295                        300
        Gly Ser Gly Gly Ser Gly Ala Glu Ala Ala Ala Lys Glu Ala Ala Ala
        305                      310                        315          320
        Lys Glu Ala Ala Ala Lys Glu Ala Ala Ala Lys Glu Ala Ala Ala Lys
                            325                      330                  335
        Glu Ala Ala Ala Lys Ala Gly Ser Gly Gly Ser Gly Gly Ser Gly Gly
                        340                      345                  350
        Ser Gly Gly Ser Gly Gly Ser Gly Gly Glu Leu Asp Arg Trp Glu Lys
                355                      360                  365
        Ile Arg Leu Arg Pro Thr Ser Gly Gly Ser Cys Gly Gly Ser Val Thr
            370                      375                  380
        Gly Tyr Arg Leu Phe Glu Glu Ile Leu Gly Gly Ser Gly Gly Ser Trp
        385                      390                  395                  400
        Ser His Pro Gln Phe Glu Lys
                        405
```

<210> 11
<211> 407
<212> PRT
<213> Artificial Sequence

<220>
<223> NB-LUMABS-6

<400> 11

```
Met Gly Ser Ser His His His His His His Ser Ser Gly Leu Val Pro
1                   5                   10                  15
Arg Gly Ser His Met Val Thr Gly Tyr Arg Leu Phe Glu Lys Glu Ser
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
    50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
            165                 170                 175
Phe Gly Arg Pro Tyr Glu Gly Ile Ala Val Phe Asp Gly Lys Lys Ile
        180                 185                 190
Thr Val Thr Gly Thr Leu Trp Asn Gly Asn Lys Ile Ile Asp Glu Arg
        195                 200                 205
Leu Ile Thr Pro Asp Gly Ser Met Leu Phe Arg Val Thr Ile Asn Ser
    210                 215                 220
Ser Gly Gly Gly Thr Glu Leu Asp Arg Trp Glu Lys Ile Arg Leu Arg
225                 230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
                275                 280                 285
Ala Ala Lys Ala Gly Ser Gly Gly Ser Gly Gly Ser Gly Gly Ser Gly
    290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Glu Leu Asp Arg Trp Glu Lys
            355                 360                 365
Ile Arg Leu Arg Pro Thr Ser Gly Gly Ser Cys Gly Gly Ser Val Thr
            370                 375                 380
Gly Tyr Arg Leu Phe Glu Lys Glu Ser Gly Gly Ser Gly Gly Ser Trp
385                 390                 395                 400
Ser His Pro Gln Phe Glu Lys
                405
```

<210> 12
<211> 407
<212> PRT
<213> Artificial Sequence

<220>
<223> NB-LUMABS-7

<400> 12

```
Met Gly Ser Ser His His His His His His Ser Ser Gly Leu Val Pro
1               5                   10                  15
Arg Gly Ser His Met Val Thr Gly Tyr Arg Leu Phe Glu Lys Glu Ser
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65              70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
        100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
        130                 135                 140
Lys Val Val Tyr Pro Val Asp Asp His His Phe Lys Val Ile Leu Pro
145             150                 155                 160
Tyr Gly Thr Leu Val Ile Asp Gly Val Thr Pro Asn Met Leu Asn Tyr
            165                 170                 175
Phe Gly Arg Pro Tyr Glu Gly Ile Ala Val Phe Asp Gly Lys Lys Ile
        180                 185                 190
Thr Val Thr Gly Thr Leu Trp Asn Gly Asn Lys Ile Ile Asp Glu Arg
        195                 200                 205
Leu Ile Thr Pro Asp Gly Ser Met Leu Phe Arg Val Thr Ile Asn Ser
        210                 215                 220
Ser Gly Gly Gly Thr Glu Leu Asp Arg Trp Glu Lys Ile Arg Leu Arg
225             230                 235                 240
Pro Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
        340                 345                 350
Ser Gly Gly Ser Gly Gly Ser Gly Gly Glu Leu Asp Arg Trp Glu Lys
        355                 360                 365
Ile Arg Leu Arg Pro Thr Ser Gly Gly Ser Cys Gly Gly Ser Val Thr
370                 375                 380
Gly Tyr Arg Leu Phe Glu Lys Ile Ser Gly Gly Ser Gly Gly Ser Trp
385                 390                 395                 400
Ser His Pro Gln Phe Glu Lys
                405
```

<210> 13

<211> 407

<212> PRT

<213> Artificial Sequence

<220>
<223> TRAS-NB-LUMABS-1

<400> 13

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Ser Gly Gly Ser Gly Gly Ser Gly
    50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
            85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
    115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Gln Leu Gly Pro Tyr Glu Leu Trp Glu Leu Ser
225                 230                 235                 240
His Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
    260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
    290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
        325                 330                 335
Glu Ala Ala Ala Lys Ala Gly Ser Gly Gly Ser Gly Gly Ser Gly Gly
        340                 345                 350
Ser Gly Gly Ser Gly Gly Ser Gly Gly Gln Leu Gly Pro Tyr Glu Leu
    355                 360                 365
Trp Glu Leu Ser His Thr Ser Gly Gly Ser Cys Gly Gly Ser Val Thr
370                 375                 380
Gly Tyr Arg Leu Phe Glu Lys Glu Ser Gly Gly Ser Gly Gly Ser Trp
385                 390                 395                 400
Ser His Pro Gln Phe Glu Lys
                405
```

<210> 14
<211> 407

<212> PRT
<213> Artificial Sequence

<220>
<223> TRAS-NB-LUMABS-2

<400> 14

```
Met Gly Ser Ser His His His His His His Ser Ser Gly Leu Val Pro
1               5                   10                  15
Arg Gly Ser His Met Val Thr Gly Tyr Arg Leu Phe Glu Lys Glu Ser
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
    50                  55                  60
Gly Ser Val Phe Thr Leu Glu Asp Phe Val Gly Asp Trp Glu Gln Thr
65              70                  75                  80
Ala Ala Tyr Asn Leu Asp Gln Val Leu Glu Gln Gly Gly Val Ser Ser
            85                  90                  95
Leu Leu Gln Asn Leu Ala Val Ser Val Thr Pro Ile Gln Arg Ile Val
        100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145             150                 155                 160
Tyr Gly Thr Leu Val Ile Asp Gly Val Thr Pro Asn Met Leu Asn Tyr
            165                 170                 175
Phe Gly Arg Pro Tyr Glu Gly Ile Ala Val Phe Asp Gly Lys Lys Ile
        180                 185                 190
Thr Val Thr Gly Thr Leu Trp Asn Gly Asn Lys Ile Ile Asp Glu Arg
        195                 200                 205
Leu Ile Thr Pro Asp Gly Ser Met Leu Phe Arg Val Thr Ile Asn Ser
    210                 215                 220
Ser Gly Gly Gly Thr Gln Leu Gly Pro Tyr Glu Leu Trp Glu Leu Ser
225                 230                 235                 240
His Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Gln Leu Gly Pro Tyr Glu Leu
        355                 360                 365
Trp Glu Leu Ser His Thr Ser Gly Gly Ser Cys Gly Gly Ser Val Thr
        370                 375                 380
Gly Tyr Arg Leu Phe Glu Lys Ile Ser Gly Gly Ser Gly Gly Ser Trp
385                 390                 395                 400
Ser His Pro Gln Phe Glu Lys
                405
```

<210> 15
<211> 407
<212> PRT
<213> Artificial Sequence

<220>
<223> TRAS-NB-LUMABS-3

<400> 15

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
            35                  40                  45
Ser Gly Ser Gly Gly Ser Gly Gly Ser Gly Gly Ser Gly Gly Ser Gly
            50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
                100                 105                 110
Arg Ser Gly Glu Asn Ala Leu Lys Ile Asp Ile His Val Ile Ile Pro
            115                 120                 125
Tyr Glu Gly Leu Ser Ala Asp Gln Met Ala Gln Ile Glu Glu Val Phe
            130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
        210                 215                 220
Ser Gly Gly Gly Thr Gln Leu Gly Pro Tyr Glu Leu Trp Glu Leu Ser
225                 230                 235                 240
His Gly Gly Ser Gly Gly Ser Gly Gly Ser Gly Gly Ser Gly Gly Ser
                245                 250                 255
Gly Gly Ser Gly Ala Glu Ala Ala Ala Lys Glu Ala Ala Ala Lys Glu
            260                 265                 270
Ala Ala Ala Lys Glu Ala Ala Ala Lys Glu Ala Ala Ala Lys Glu Ala
```

```
                275                         280                         285
        Ala Ala Lys Ala Gly Ser Gly Gly Ser Gly Gly Ser Gly Gly Ser Gly
            290                         295                         300
        Gly Ser Gly Gly Ser Gly Ala Glu Ala Ala Ala Lys Glu Ala Ala Ala
        305                         310                         315                         320
        Lys Glu Ala Ala Ala Lys Glu Ala Ala Ala Lys Glu Ala Ala Ala Lys
                    325                         330                         335
        Glu Ala Ala Ala Lys Ala Gly Ser Gly Gly Ser Gly Gly Ser Gly Gly
                    340                         345                         350
        Ser Gly Gly Ser Gly Gly Ser Gly Gly Gln Leu Gly Pro Tyr Glu Leu
                    355                         360                         365
        Trp Glu Leu Ser His Thr Ser Gly Gly Ser Cys Gly Gly Ser Val Thr
                    370                         375                         380
        Gly Tyr Arg Leu Phe Glu Glu Ile Leu Gly Gly Ser Gly Gly Ser Trp
        385                         390                         395                         400
        Ser His Pro Gln Phe Glu Lys
                    405
```

<210> 16
<211> 56
<212> PRT
<213> streptococcus

<220>
<223> Protein G

<220>
<221> BINDING
<222> 24
<223> photocrosslinker p-benzoyl-1-phenylalanine (BPA)

<400> 16

```
        Met Thr Phe Lys Leu Ile Ile Asn Gly Lys Thr Leu Lys Gly Glu Ile
        1                   5                           10                          15
        Thr Ile Glu Ala Val Asp Ala Xaa Glu Ala Glu Lys Ile Phe Lys Gln
                    20                          25                          30
        Tyr Ala Asn Asp Tyr Gly Ile Asp Gly Glu Trp Thr Tyr Asp Asp Ala
                    35                          40                          45
        Thr Lys Thr Phe Thr Val Thr Glu
                50                          55
```

<210> 17
<211> 17
<212> PRT
<213> Artificial Sequence

<220>
<223> Anti-CRP169 binding epitope

<400> 17

```
        Val Gly Ala Glu Ala Ser Ile Ile Leu Gly Gln Glu Gln Asp Ser Phe
        1                   5                           10                          15
        Gly
```

<210> 18
<211> 17
<212> PRT

<213> Artificial Sequence

<220>
<223> Anti-CRP36 binding epitope

<400> 18

```
Tyr Ser Phe Thr Val Gly Gly Ser Glu Ile Leu Phe Glu Val Pro Glu
1               5                   10                  15
Val
```

<210> 19
<211> 158
<212> PRT
<213> Oplophorus gracilirostris

<220>
<223> LBiT

<400> 19

```
Val Phe Thr Leu Glu Asp Phe Val Gly Asp Trp Glu Gln Thr Ala Ala
1               5                   10                  15
Tyr Asn Leu Asp Gln Val Leu Glu Gln Gly Gly Val Ser Ser Leu Leu
            20                  25                  30
Gln Asn Leu Ala Val Ser Val Thr Pro Ile Gln Arg Ile Val Arg Ser
            35                  40                  45
Gly Glu Asn Ala Leu Lys Ile Asp Ile His Val Ile Ile Pro Tyr Glu
    50                  55                  60
Gly Leu Ser Ala Asp Gln Met Ala Gln Ile Glu Glu Val Phe Lys Val
65                  70                  75                  80
Val Tyr Pro Val Asp Asp His His Phe Lys Val Ile Leu Pro Tyr Gly
                85                  90                  95
Thr Leu Val Ile Asp Gly Val Thr Pro Asn Met Leu Asn Tyr Phe Gly
                100                 105                 110
Arg Pro Tyr Glu Gly Ile Ala Val Phe Asp Gly Lys Lys Ile Thr Val
            115                 120                 125
Thr Gly Thr Leu Trp Asn Gly Asn Lys Ile Ile Asp Glu Arg Leu Ile
    130                 135                 140
Thr Pro Asp Gly Ser Met Leu Phe Arg Val Thr Ile Asn Ser
145                 150                 155
```

<210> 20
<211> 120
<212> PRT
<213> Artificial Sequence

<220>
<223> semi-flexible linker

<400> 20

```
Gly Gly Ser Gly Gly Ser Gly Gly Ser Gly Gly Ser Gly Gly Ser Gly
1               5                   10              15
Gly Ser Gly Ala Glu Ala Ala Ala Lys Glu Ala Ala Ala Lys Glu Ala
        20                  25                  30
Ala Ala Lys Glu Ala Ala Ala Lys Glu Ala Ala Ala Lys Glu Ala Ala
        35                  40                  45
Ala Lys Ala Gly Ser Gly Gly Ser Gly Gly Ser Gly Gly Ser Gly Gly
    50                  55                  60
Ser Gly Gly Ser Gly Ala Glu Ala Ala Ala Lys Glu Ala Ala Ala Lys
65                  70                  75                  80
Glu Ala Ala Ala Lys Glu Ala Ala Ala Lys Glu Ala Ala Ala Lys Glu
                85                  90                  95
Ala Ala Ala Lys Ala Gly Ser Gly Gly Ser Gly Gly Ser Gly Gly Ser
                100                 105                 110
Gly Gly Ser Gly Gly Ser Gly Gly
        115                 120
```

<210> 21
<211> 462
<212> PRT
<213> Artificial Sequence

<220>
<223> pG-NB-LUMABS-17

<220>
<221> BINDING
<222> 254
<223> photocrosslinker p-benzoyl-1-phenylalanine (BPA)

<400> 21

```
Met Gly Ser Ser His His His His His His Ser Ser Gly Leu Val Pro
1               5                   10              15
Arg Gly Ser His Met Val Thr Gly Tyr Arg Leu Phe Glu Glu Ile Leu
        20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
            115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
210                 215                 220
Gly Gly Ser Gly Gly Ser Met Thr Phe Lys Leu Ile Ile Asn Gly Lys
225                 230                 235                 240
Thr Leu Lys Gly Glu Ile Thr Ile Glu Ala Val Asp Ala Xaa Glu Ala
            245                 250                 255
Glu Lys Ile Phe Lys Gln Tyr Ala Asn Asp Tyr Gly Ile Asp Gly Glu
            260                 265                 270
Trp Thr Tyr Asp Asp Ala Thr Lys Thr Phe Thr Val Thr Glu Gly Thr
    275                 280                 285
Gly Gly Ser Gly Gly Ser Gly Gly Ser Gly Gly Ser Gly Gly Ser Gly
    290                 295                 300
Gly Ser Gly Ala Glu Ala Ala Ala Lys Glu Ala Ala Ala Lys Glu Ala
305                 310                 315                 320
Ala Ala Lys Glu Ala Ala Ala Lys Glu Ala Ala Ala Lys Glu Ala Ala
            325                 330                 335
Ala Lys Ala Gly Ser Gly Gly Ser Gly Gly Ser Gly Gly Ser Gly Gly
            340                 345                 350
```

```
Ser Gly Gly Ser Gly Ala Glu Ala Ala Ala Lys Glu Ala Ala Ala Lys
    355                 360                 365
Glu Ala Ala Ala Lys Glu Ala Ala Ala Lys Glu Ala Ala Ala Lys Glu
    370                 375                 380
Ala Ala Ala Lys Ala Gly Ser Gly Gly Ser Gly Gly Ser Gly Gly Ser
385                 390                 395                 400
Gly Gly Ser Gly Gly Ser Gly Gly Val Gly Ala Glu Ala Ser Ile Ile
            405                 410                 415
Leu Gly Gln Glu Gln Asp Ser Phe Gly Gly Gly Ser Gly Gly Ser Cys
            420                 425                 430
Thr Ser Gly Gly Ser Val Thr Gly Tyr Arg Leu Phe Glu Lys Glu Ser
            435                 440                 445
Gly Gly Ser Gly Gly Ser Trp Ser His Pro Gln Phe Glu Lys
    450                 455                 460
```

<210> 22
<211> 462
<212> PRT
<213> Artificial Sequence

<220>
<223> pG-NB-LUMABS-11

<220>
<221> BINDING
<222> 254
<223> photocrosslinker p-benzoyl-1-phenylalanine (BPA)

<400> 22

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
                20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
    50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
            115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Gly Gly Ser Gly Gly Ser Met Thr Phe Lys Leu Ile Ile Asn Gly Lys
225                 230                 235                 240
Thr Leu Lys Gly Glu Ile Thr Ile Glu Ala Val Asp Ala Xaa Glu Ala
            245                 250                 255
Glu Lys Ile Phe Lys Gln Tyr Ala Asn Asp Tyr Gly Ile Asp Gly Glu
```

```
                          260                     265                     270
         Trp Thr Tyr Asp Asp Ala Thr Lys Thr Phe Thr Val Thr Glu Gly Thr
                      275                     280                     285
         Gly Gly Ser Gly Gly Ser Gly Gly Ser Gly Gly Ser Gly Gly Ser Gly
                      290                     295                     300
         Gly Ser Gly Ala Glu Ala Ala Ala Lys Glu Ala Ala Ala Lys Glu Ala
         305                     310                     315                     320
         Ala Ala Lys Glu Ala Ala Ala Lys Glu Ala Ala Ala Lys Glu Ala Ala
                              325                     330                     335
         Ala Lys Ala Gly Ser Gly Gly Ser Gly Gly Ser Gly Gly Ser Gly Gly
                      340                     345                     350
         Ser Gly Gly Ser Gly Ala Glu Ala Ala Ala Lys Glu Ala Ala Ala Lys
                      355                     360                     365
         Glu Ala Ala Ala Lys Glu Ala Ala Ala Lys Glu Ala Ala Ala Lys Glu
                      370                     375                     380
         Ala Ala Ala Lys Ala Gly Ser Gly Gly Ser Gly Gly Ser Gly Gly Ser
         385                     390                     395                     400
         Gly Gly Ser Gly Gly Ser Gly Gly Tyr Ser Phe Thr Val Gly Gly Ser
                              405                     410                     415
         Glu Ile Leu Phe Glu Val Pro Glu Val Gly Gly Ser Gly Gly Ser Cys
                      420                     425                     430
         Thr Ser Gly Gly Ser Val Thr Gly Tyr Arg Leu Phe Glu Lys Glu Ser
                      435                     440                     445
         Gly Gly Ser Gly Gly Ser Trp Ser His Pro Gln Phe Glu Lys
                      450                     455                     460
```

<210> 23
<211> 12
<212> PRT
<213> Artificial Sequence

<220>
<223> Cetuximab binding meditope with monovalent K(d) of 61 nM

<400> 23

```
                   Cys Val Phe Asp Leu Gly Thr Arg Arg Leu Arg Cys
                   1               5                   10
```

<210> 24
<211> 12
<212> PRT
<213> Artificial Sequence

<220>
<223> Cetuximab binding meditope with monovalent Kd of 270 nM

<400> 24

```
                   Cys Gln Phe Asp Leu Ser Thr Arg Arg Leu Lys Cys
                   1               5                   10
```

<210> 25
<211> 407
<212> PRT
<213> Artificial Sequence

<220>
<223> CTX-NB-LUMABS-1

<220>
<221> BINDING
<222> 379
<223> photocrosslinker p-azido-1-phenylalanine (pAzF)

<400> 25

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
        115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
            165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Cys Val Phe Asp Leu Gly Thr Arg Arg Leu Arg
225                 230                 235                 240
Cys Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
            325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Cys Val Phe Asp Leu Gly Thr
            355                 360                 365
Arg Arg Leu Arg Cys Thr Ser Gly Gly Ser Xaa Gly Gly Ser Val Thr
    370                 375                 380
Gly Tyr Arg Leu Phe Glu Lys Glu Ser Gly Gly Ser Gly Gly Ser Trp
385                 390                 395                 400
Ser His Pro Gln Phe Glu Lys
                405
```

<210> 26

<211> 407
<212> PRT
<213> Artificial Sequence

<220>
<223> CTX-NB-LUMABS-2

<220>
<221> BINDING
<222> 396
<223> photocrosslinker p-azido-1-phenylalanine (pAzF)

<400> 26

```
Met Gly Ser Ser His His His His His His Ser Ser Gly Leu Val Pro
1               5                   10              15
Arg Gly Ser His Met Val Thr Gly Tyr Arg Leu Phe Glu Glu Ile Leu
            20              25              30
Gly Ser Gly Gly Ser Gly Gly Ser Gly Gly Ser Gly Gly Ser Gly Gly
        35              40              45
Ser Gly Ser Gly Gly Ser Gly Gly Ser Gly Gly Ser Gly Gly Ser Gly
    50              55              60
Gly Ser Val Phe Thr Leu Glu Asp Phe Val Gly Asp Trp Glu Gln Thr
65              70              75              80
Ala Ala Tyr Asn Leu Asp Gln Val Leu Glu Gln Gly Gly Val Ser Ser
            85              90              95
Leu Leu Gln Asn Leu Ala Val Ser Val Thr Pro Ile Gln Arg Ile Val
        100             105             110
Arg Ser Gly Glu Asn Ala Leu Lys Ile Asp Ile His Val Ile Ile Pro
    115             120             125
Tyr Glu Gly Leu Ser Ala Asp Gln Met Ala Gln Ile Glu Glu Val Phe
    130             135             140
Lys Val Val Tyr Pro Val Asp Asp His His Phe Lys Val Ile Leu Pro
145             150             155             160
Tyr Gly Thr Leu Val Ile Asp Gly Val Thr Pro Asn Met Leu Asn Tyr
            165             170             175
Phe Gly Arg Pro Tyr Glu Gly Ile Ala Val Phe Asp Gly Lys Lys Ile
        180             185             190
Thr Val Thr Gly Thr Leu Trp Asn Gly Asn Lys Ile Ile Asp Glu Arg
        195             200             205
Leu Ile Thr Pro Asp Gly Ser Met Leu Phe Arg Val Thr Ile Asn Ser
    210             215             220
Ser Gly Gly Gly Thr Cys Val Phe Asp Leu Gly Thr Arg Arg Leu Arg
225             230             235             240
Cys Gly Gly Ser Gly Gly Ser Gly Gly Ser Gly Gly Ser Gly Gly Ser
            245             250             255
Gly Gly Ser Gly Ala Glu Ala Ala Ala Lys Glu Ala Ala Ala Lys Glu
        260             265             270
Ala Ala Ala Lys Glu Ala Ala Ala Lys Glu Ala Ala Ala Lys Glu Ala
    275             280             285
Ala Ala Lys Ala Gly Ser Gly Gly Ser Gly Gly Ser Gly Gly Ser Gly
    290             295             300
Gly Ser Gly Gly Ser Gly Ala Glu Ala Ala Ala Lys Glu Ala Ala Ala
305             310             315             320
Lys Glu Ala Ala Ala Lys Glu Ala Ala Ala Lys Glu Ala Ala Ala Lys
            325             330             335
Glu Ala Ala Ala Lys Ala Gly Ser Gly Gly Ser Gly Gly Ser Gly Gly
        340             345             350
Ser Gly Gly Ser Gly Gly Ser Gly Gly Cys Val Phe Asp Leu Gly Thr
    355             360             365
Arg Arg Leu Arg Cys Thr Ser Gly Gly Ser Gly Gly Ser Val Thr Gly
    370             375             380
Tyr Arg Leu Phe Glu Lys Glu Ser Gly Gly Ser Xaa Gly Gly Ser Trp
385             390             395             400
Ser His Pro Gln Phe Glu Lys
            405
```

<210> 27

<211> 407

<212> PRT

<213> Artificial Sequence

<220>

<223> CTX-NB-LUMABS-3

<220>
<221> BINDING
<222> 379
<223> photocrosslinker p-azido-1-phenylalanine (pAzF)

<400> 27

```
Met Gly Ser Ser His His His His His His Ser Ser Gly Leu Val Pro
1               5                   10                  15
Arg Gly Ser His Met Val Thr Gly Tyr Arg Leu Phe Glu Glu Ile Leu
            20                  25                  30
Gly Ser Gly Gly Ser Gly Gly Ser Gly Gly Ser Gly Gly Ser Gly Gly
        35                  40                  45
Ser Gly Ser Gly Gly Ser Gly Gly Ser Gly Gly Ser Gly Gly Ser Gly
        50                  55                  60
Gly Ser Val Phe Thr Leu Glu Asp Phe Val Gly Asp Trp Glu Gln Thr
65                  70                  75                  80
Ala Ala Tyr Asn Leu Asp Gln Val Leu Glu Gln Gly Gly Val Ser Ser
                85                  90                  95
Leu Leu Gln Asn Leu Ala Val Ser Val Thr Pro Ile Gln Arg Ile Val
            100                 105                 110
Arg Ser Gly Glu Asn Ala Leu Lys Ile Asp Ile His Val Ile Ile Pro
            115                 120                 125
Tyr Glu Gly Leu Ser Ala Asp Gln Met Ala Gln Ile Glu Glu Val Phe
    130                 135                 140
Lys Val Val Tyr Pro Val Asp Asp His His Phe Lys Val Ile Leu Pro
145                 150                 155                 160
Tyr Gly Thr Leu Val Ile Asp Gly Val Thr Pro Asn Met Leu Asn Tyr
                165                 170                 175
Phe Gly Arg Pro Tyr Glu Gly Ile Ala Val Phe Asp Gly Lys Lys Ile
            180                 185                 190
Thr Val Thr Gly Thr Leu Trp Asn Gly Asn Lys Ile Ile Asp Glu Arg
            195                 200                 205
Leu Ile Thr Pro Asp Gly Ser Met Leu Phe Arg Val Thr Ile Asn Ser
    210                 215                 220
Ser Gly Gly Gly Thr Cys Gln Phe Asp Leu Ser Thr Arg Arg Leu Lys
225                 230                 235                 240
Cys Gly Gly Ser Gly Gly Ser Gly Gly Ser Gly Gly Ser Gly Gly Ser
            245                 250                 255
Gly Gly Ser Gly Ala Glu Ala Ala Ala Lys Glu Ala Ala Ala Lys Glu
        260                 265                 270
Ala Ala Ala Lys Glu Ala Ala Ala Lys Glu Ala Ala Ala Lys Glu Ala
        275                 280                 285
Ala Ala Lys Ala Gly Ser Gly Gly Ser Gly Gly Ser Gly Gly Ser Gly
        290                 295                 300
Gly Ser Gly Gly Ser Gly Ala Glu Ala Ala Ala Lys Glu Ala Ala Ala
305                 310                 315                 320
Lys Glu Ala Ala Ala Lys Glu Ala Ala Ala Lys Glu Ala Ala Ala Lys
                325                 330                 335
Glu Ala Ala Ala Lys Ala Gly Ser Gly Gly Ser Gly Gly Ser Gly Gly
            340                 345                 350
Ser Gly Gly Ser Gly Gly Ser Gly Gly Cys Gln Phe Asp Leu Ser Thr
    355                 360                 365
Arg Arg Leu Lys Cys Thr Ser Gly Gly Ser Xaa Gly Gly Ser Val Thr
370                 375                 380
Gly Tyr Arg Leu Phe Glu Lys Glu Ser Gly Gly Ser Gly Gly Ser Trp

385                 390                 395                 400
Ser His Pro Gln Phe Glu Lys
                405
```

**Description**

Field of the invention

The invention relates to a biosensor and a detection method, wherein the biosensor is used for detecting and quantifying biomolecules (e.g. antibodies, antigens, proteins, etc.) or ligands (e.g. small molecules, protein based biomarkers, etc.). In particular, this invention relates to a bioluminescent biosensor and a detection method, wherein the bioluminescent biosensor is used for detecting and quantifying antibodies. The invention further relates to a biosensor and a detection method, wherein the biosensor is a biomolecule (e.g. an antibody) conjugated biosensor detecting and quantifying ligands, such as small molecules or protein based biomarkers.

Background

Antibody-based molecular recognition plays a dominant role in the life sciences ranging from applications in diagnostics and molecular imaging to targeted drug delivery and therapy. Antibodies are important biomarkers in a broad range of diseases, and especially important for the diagnosis and surveillance of infectious diseases, autoimmune diseases, and allergies. In addition, antibody-based drug therapies constitute an important class of biomolecular drugs. E.g. therapeutic antibodies are clinically applied in anticancer and anti-inflammatory therapy. The initial response and clearance rates of therapeutic antibodies vary per individual patient and correlate with treatment efficacy and disease progression, suggesting that patient-specific therapeutic drug monitoring (TDM) would allow better efficacy by preventing both over- and under-dosing. The detection and quantification of antibodies remains an important need in point-of-care testing. While a wide variety of sensitive antibody detection strategies have been developed, many of them come with intrinsic limitations such as the requirement for multiple time-consuming incubation steps (ELISA and other heterogeneous, sandwich-type assays), multiple reagents, and/or sophisticated equipment (e.g. surface plasmon resonance).

New generic antibody detection strategies in which molecular recognition and signal generation are integrated within a single protein would be ideal, in particular for high-throughput screening and point-of-care applications. From a protein engineering perspective, the key question is then how antibody binding can be translated into a readily detectable signal change.

One approach is to make use of fluorescently labeled epitopes whose fluorescence properties are changed upon antibody binding. However, due to lack of enzyme amplification step, the sensitivity of these methods is limited by the concentration of fluorescent probe that can be reliably detected. An additional drawback is the requirement of specialized instrumentation which is particularly problematic if diagnosis is required in low-resource areas or outside medical centers.

Several groups have reported the development of allosteric antibody reporter enzyme by inserting peptide epitopes at permissive sites within reporter enzyme (see: Brennan, et al. Protein Eng., 1994, 7, 509; Benito, et al. J. Biol. Chem., 1996, 271, 21251; and Ferrer-Miralles, et al. Journal of Biological Chemistry, 2001, 276, 40087). However, these hybrid enzymes are catalytically compromised and analyte binding often results in a further decrease in activity. A different design strategy is to make use of antibody-induced oligomerization of reporter enzymes or complementation of split reporter enzymes (see: Geddie, et al. J. Mol. Biol., 2007, 369, 1052; De las Heras, et al. Biochem. Biophys. Res. Commun., 2008, 370, 164; and Fry, et al. Biochem. Biophys. Res. Commun., 2008, 372, 542). These approaches utilize the bivalent nature of antibodies to bring together two proteins (or protein domains) to form an active enzyme. However, the reconstituted enzyme activity is typically low (only 1-2%) compared to its parent enzyme. An additional drawback is that the sensor performance is dependent on the sensor concentrations and therefore less robust than a single protein sensor.

Instead of using antibody to bring together a two-component reporter system, antibody binding can also be used to disrupt the interaction between two domains. The advantage of this strategy is that the two domains can be part of a single protein construct. Proof of principle was provided by Merkx's group (Golynskiy, et al. ChemBioChem, 2010, 11, 2264) in the design of a protein-based antibody sensor based on the principle of Forster Resonance Energy transfer (FRET). Two fluorescent proteins were tethered together via a semi-flexible linker that contained two identical epitopes adjacent to the fluorescent domains. The linker contained two α-helical blocks to ensure efficient bridging of the about 10 nm distance between the antigen binding domains of the target antibody. Because the fluorescent proteins contained self-association promoting mutations, they formed an intramolecular domain interaction in the absence of antibody. Bivalent binding of an antibody to the epitopes in the linker disrupted the interaction between the fluorescent domains, resulting in a decrease in FRET. The limitation of detection for this FRET sensor was that detection relied on external illumination and lacked a signal amplification step. The same molecular switch principle was used by the group to control an intramolecular interaction between a reporter enzyme and its inhibitor protein, allowing enzyme-amplified detection of picomolar antibody concentrations directly in solution (Banala, et al. ACS Chem. Biol., 2013, 8, 2127). However, the reporter enzyme showed attenuated activity in serum and it lacked the intrinsic calibration provided by the dual-color FRET system. The group further utilized the same principle to design a sensor format (named LUMABS) based on bioluminescence resonance energy transfer (BRET) (Arts, et al. Anal. Chem., 2016, 88, 4525). The blue-light emitting luciferase (e.g. NanoLuc; also referred to as 'NLuc') was used as a donor and connected via a semi-flexible linker to the green fluorescent acceptor protein (e.g. mNeonGreen) and a helper domain was introduced to bring them in close proximity. Bivalent binding of an antibody flanking the linker disrupted the interaction between the helper domains, resulting in a decrease of BRET and thus a change of the luminescence color from green to blue. A key advantage of bioluminescent sensors is that they do not require external excitation and thus do not suffer from background fluorescence and scattering, allowing sensitive detection of the sensor signal against essentially a dark background. More recently, the Merkx's group reported the construction of LUMABS sensors specifically targeting the therapeutic antibodies trastuzumab and cetuximab that contains disulfide-containing cyclic peptide epitopes (Van Rosmalen, et al. Anal. Chem., 2018, DOI: 10.1021/acs.analchem.8b00041) and the development of semisynthetic LUMABS in which the introduction of the non-natural amino-acid *p*-azidophenylalanine allowed the conjugation of non-peptide antigens (Arts, et al. ACS Sens., 2017, 2, 1730).

NanoLuc, an engineered luciferase derived from a deep-sea shrimp, is a small (19 kDa) but stable protein which produces increased and sustained luminescence compared to other luciferases (Hall, M.P., et al. ACS Chem Biol, 2012, 7, 1848). NanoLuc thus represents an attractive donor for BRET-based assays. The LUMABS sensor developed by Merkx's group (Arts, et al. Anal. Chem., 2016, 88, 452) utilized NanoLuc as a donor and mNeonGreen as acceptor whose emission peaks are separated by only 56 nm. The large overlap of emission spectra means that part of the observed intensity at mNeonGreen's emission peak originates from NanoLuc, which limits the maximal ratiometric response. A more red-shifted fluorescent acceptor would be a good alternative due to the enhanced separation between the emission spectra and thus a large change in emission ratio between the antibody-free and -bound state might be achieved. However, replacement of mNeonGreen by red fluorescent protein domains yielded LUMABS sensors with a poor dynamic range, a result of inefficient BRET between NanoLuc and the red-shifted fluorescent acceptor (Hall, et al. ACS Chem. Biol., 2012, 7, 1848).

Split luciferase is an effective tool for examination of protein interactions and for detection of analytes both *in vivo* and *in vitro.* For assaying analytes, a domain or an intact protein is usually inserted into an internally fragmented luciferase, resulting in ligand binding, which causes a change in the emitted signals. Recently, NanoLuc was split into two fragments, an 18 kDa large bit (LBiT) and a 1.3 kDa small bit (SBiT) and designed as a complementation reporter (named NanoBiT, or NB) for the study of protein interactions (Dixon, et al. ACS Chem. Biol., 2016, 11, 400). The NanoBiT (NB) protein complementation assay have been utilized for analysis of G protein interactions (Christopher, et al. Anal. Biochem., 2017, 522, 10) and viral entry and release (Sasaki, et al. Virus Res., 2018, 243, 69). A tri-part NanoBiT complementation system based on two small peptide-fusions was recently developed for homogeneous immunoassay (Dixon, et al. Sci Rep., 2017, 7, 8186).

The present invention provides a different bioluminescent sensor format that allows direct detection of analytes in particular antibodies in solution based on mutually exclusive intramolecular split luciferase complementation and addresses many of the limitations described *supra.*

Summary of the invention

Within this invention we present a new approach that allows one-step detection of biomolecules (e.g. antibodies, proteins, antigens, aptamers or the like) or biomolecules specific ligands (e.g. small molecules, protein biomarkers or the like) directly in solution using intramolecular complementation of split luciferase. The present invention provides hereto a biosensor, preferably a ratiometric biosensor, including a linker comprising a first end and a second end, wherein the first end of the linker is fused to one terminus of a first luciferase domain via a first binding domain and the second end of the linker is fused to a second luciferase domain via a second binding domain. The biosensor optionally comprises a third luciferase domain fused to the other terminus of the first luciferase domain via a spacer. The binding domains of the biosensor are configured to bind to a biomolecule, wherein the biosensor exists in at least two conformations, in which either the second or third luciferase domain binds to the first luciferase domain to form a complemented luciferase. By providing a biosensor wherein the second and third luciferase domains bind to the first luciferase domain to form a complemented luciferase, and wherein a fluorophore is conjugated next to one of the second or third luciferase domains an efficient Bioluminescence Resonance Energy Transfer (BRET) between the luciferase and the fluorophore in only one of the two conformations is provided. It is noted that the third luciferase domain may be fused to the first luciferase domain, however may alternatively be present as a component in, for example, a solution comprising the biosensor of the invention.

The term "biomolecule" or "biological molecule" as used herein refers to a molecule that is present in organisms, essential to some typically biological process such as cell division, morphogenesis, or development, and may include, but not limited to, large macromolecules such as antibodies, antigens, aptamers, proteins, carbohydrates, lipids, and nucleic acids (RNA and DNA fragments), as well as small molecules such as primary metabolites, secondary metabolites, and natural products. Biomolecules are usually endogenous but may also be exogenous. For example, pharmaceutical drugs may be natural products or semisynthetic (biopharmaceuticals) or they may be totally synthetic.

The term "ligand" as used herein refers to a substance that forms a complex with a biomolecule to serve a biological purpose. In protein-ligand binding, the ligand is typically a molecule which produces a signal by binding to a site on a target protein. In DNA-ligand binding studies, the ligand can be a small molecule, ion, protein or the like. Typically, binding between the ligand and the biomolecule occurs by intermolecular forces, such as ionic bonds, hydrogen bonds and Van der Waals forces. The association of docking is actually reversible through dissociation.

The term "via" (as for example use in the context of "via a first binding domain") as used herein refers to a configuration wherein a first part of the biosensor is fused to a second part of the biosensor with the interposition of (via) a third part of the biosensor, wherein that third part of the biosensor itself is connected to both first and second part of the biosensor or wherein that third part is connected to a linking part of the biosensor linking the first and second part of the biosensor.

The term "luciferase domain" as used herein refers to oxidative enzymes, including moieties or fragments thereof that produce bioluminescence. Examples include (but are not limited to) split firefly luciferase and NanoLuc, an engineered luciferase derived from a deep-sea shrimp. The luciferase domain may comprise fragments of a luciferase, preferably fragments of a split luciferase, which complemented fragments forms a complemented luciferase having luciferase activity. An example of such fragments include the NanoLuc large split luciferase fragment and NanoLuc small split luciferase fragment.

Optionally, the luciferase domains are protein domains or polypeptides capable of self-assembly into a bioluminescent complex. Optionally, the luciferase domains are split NanoLuc fragments. Optionally, the first luciferase domain is a large luciferase domain, preferably a large split luciferase fragment. Further optionally, the second and third luciferase domains are small luciferase domains, preferably small split luciferase fragments.

Optionally, the first luciferase domain is selected from an amino acid sequence of SEQ ID NO: 19, or having an identity of at least 70%, 75%, 80%, 85%, 90%, 95%, 96 % , 97 %, 98 %, or 99 % to the amino acid sequence of SEQ ID NO: 19.

Optionally, each of the second and third luciferase domains is selected from the group of amino acid sequences consisting of SEQ ID NO: 1, SEQ ID NO: 2 or SEQ ID NO: 3, or having an identity of at least 70%, 75%, 80%, 85%, 90%, 95%, 96 % , 97 %, 98 %, or 99 % to the amino acid sequences of SEQ ID NO: 1, SEQ ID NO: 2 or SEQ ID NO: 3.

In an embodiment of the invention, the affinity of the second and third luciferase domains to the first luciferase domain differs. Preferably, the second luciferase domain has a higher affinity compared to the affinity of the third luciferase domain.

Optionally, the affinity of the second luciferase domain has an affinity which is at least 5 fold higher, preferably between 10 and 1500 fold higher, between 10 and 1000 fold higher, between 10 and 100 fold higher or between 10 and 50 fold higher compared to the affinity of the third luciferase domain.

In an embodiment of the invention, the third luciferase domain is fused to the other terminus of the first luciferase domain via a spacer.

The terms "linker" and "spacer" as used herein refer to a linker suitable for linking the second luciferase domain and, optionally, the third luciferase domain to the first luciferase domain. The length of the linker and the optional spacer depends on the linking position of the linker and the optional spacer to the first luciferase domain (whether it is linked to the N-terminus or C-terminus of the first luciferase domain) in combination with the position of the binding site of the first luciferase domain. The content of the linker and the optional spacer depends on the desired flexibility of the linker and the optional spacer. The desired flexibility may vary per biosensor and per purpose.

Optionally, the spacer is a polypeptide or polypeptide fragment and may include at least four, at least six, at least eight or at least ten GGS repeats. However, it is noted that the number of GGS repeats may vary depending on the purpose and biosensor design. Even the content of the linker may vary and is not necessarily restricted to the use of GGS repeats.

Optionally, the linker is a semi-flexible linker such as a polypeptide or polypeptide fragment and may include at least one flexible block of $(GSG)_6$. Further optionally, the linker further includes at least one $\alpha$-helical block. Still further optionally, the linker further includes at least one $\alpha$-helical block having six EAAAK repeats. Still further optionally, the linker further includes two $\alpha$-helical blocks, each having six EAAAK repeats. In general it is noted that the semi-flexible linker is chosen such that the linker has enough flexibility to change the state of the biosensor, i.e. changing the BRET between the luciferase and the fluorophore in the two conformations.

Optionally, the semi-flexible linker is selected from an amino acid sequence of SEQ ID NO: 20, or having an identity of at least 70%, 75%, 80%, 85%, 90%, 95%, 96 % , 97 %, 98 %, or 99 % to the amino acid sequence of SEQ ID NO: 20.

Optionally, the fluorophore is a fluorescent chemical compound that can emit red light upon light excitation. Further optionally, the fluorophore is Cy3.

Optionally, the fluorophore is conjugated to the biosensor adjacent to the second luciferase domain. Further optionally, the fluorophore is conjugated to the biosensor between the linker and second luciferase domain. Alternatively, the fluorophore is conjugated to the biosensor adjacent to the third luciferase domain.

Optionally, the biomolecule may comprise an antibody, antigen, protein or aptamer. Preferably, the biomolecule is an antibody.

In a first aspect of the invention, both binding domains are configured to bind with the biomolecule, i.e. to competitively bind to the one or more binding sites of the biomolecule. Although the binding domains may be any kind of binding domain having a certain affinity with the binding site or binding sites of the biomolecule, the use of a binding domain being an epitope is preferred. Optionally, both binding domains comprise the same epitopes, i.e. epitopes configured to bind intermolecular with both paratopes of an antibody.

The term "epitope" as used herein refers to a polypeptide which bind to the antigen-binding site of an antibody, i.e. the paratope of an antibody. The term "epitope" as used herein is not limited to natural existing epitopes per se. The term "epitope" as used herein may include epitope mimicking mimotopes or epitope-like antibody binding peptides including meditopes or the like, such as aptamers and small molecules.

The term "meditopes" as used herein refers to peptides that specifically recognize an antibody by binding in a cavity at the interface between the constant and variable domains. Examples of meditopes are disclosed in, for example, Van Rosmalen (Anal. Chem., 2018, 90, 3592).

Optionally, the epitope is varied based on the biomolecule (e.g. antibody) to be detected. For example, for the detection of anti-HIV-p17 antibodies the two epitopes may be selected from an amino acid sequence of SEQ ID NO: 4, or having an identity of at least 70%, 75%, 80%, 85%, 90%, 95%, 96 % , 97 %, 98 %, or 99 % to the amino acid sequence of SEQ ID NO: 4. Alternatively, for the detection of trastuzumab the two epitopes may comprise a mimotope and may be selected from an amino acid sequence of SEQ ID NO: 5, or having an identity of at least 70%, 75%, 80%, 85%, 90%, 95%, 96 % , 97 %, 98 %, or 99 % to the amino acid sequence of SEQ ID NO: 5. Further alternatively, for the detection of cetuximab the two epitopes may comprise a meditope and may be selected from an amino acid sequence of SEQ ID NO: 23 or SEQ ID NO: 24, or having an identity of at least 70%, 75%, 80%, 85%, 90%, 95%, 96 % , 97 %, 98 %, or 99 % to the amino acid sequence of SEQ ID NO: 23 or SEQ ID NO: 24. Even further alternatively, for the detection of anti-C-reactive protein (CRP) antibody the two epitopes may be selected from an amino acid sequence of SEQ ID NO: 17 (for detecting anti-CRP169 antibody) or SEQ ID NO: 18 (for detecting anti-CRP36 antibody), or having an identity of at least 70%, 75%, 80%, 85%, 90%, 95%, 96 % , 97 %, 98 %, or 99 % to the amino acid sequence of SEQ ID NO: 17 or SEQ ID NO: 18.

In this first aspect of the invention, the invention further relates to an *in vitro* biomolecule-detecting method, comprising the steps of:

- contacting a sample with the biosensor of the first aspect of the invention;
- determining the change of the biosensor's luminescence in the presence of a sample; and
- determining the luminescence change in the presence of the sample to the quantitative and/or qualitative presence or absence of a biomolecule.

By providing a biosensor having an interacting first and second luciferase domain in further combination with a third luciferase domain optionally linked to the first luciferase domain, the method of the present invention provides the possibility to determine the luminescence change in the presence of the sample in order to define the quantitative and/or qualitative presence or absence of an biomolecule.

Optionally, the invention relates to a method wherein in the absence of a biomolecule the biosensor is in a biomolecule-free (closed) state that at least some of the first luciferase domain complements with the second luciferase domain. In case the fluorophore is next to the second luciferase domain, in the biomolecule-free state the fluorophore is in close proximity to the complemented luciferase formed by the first luciferase domain and second luciferase domain.

Optionally, the invention relates to a method wherein, in the presence of a biomolecule, binding between the binding domains of the biosensor and the biomolecule changes the equilibrium between the biomolecule-free (closed) state of the biosensor and a biomolecule-bound (open) state of the biosensor such that the BRET between the luciferase and the fluorophore changes. In case the fluorophore is next to the second luciferase domain, the BRET between the luciferase and the fluorophore decreases in the biomolecule-bound state compared to the biosensor in the biomolecule-free state.

In a second aspect of the invention, the first binding domain is configured to form a conjugated system with the biomolecule. In such configuration, the second binding domain is configured to bind to a ligand binding site of the biomolecule. Optionally, the biomolecule is covalently bound to the first binding domain of the biosensor. Further optionally, the biomolecule comprises an antibody wherein the Fc-region of the antibody covalently binds to the first binding domain of the biosensor.

The terms "conjugate system", "conjugation", "conjugate binding" and the like as used herein refer to a binding state of the biosensor of the invention and the biomolecule wherein the affinity between the biosensor and the biomolecule is selected such that the ligand binding to the biomolecule and any other component present in the sample to be analysed do not compete with the binding of the conjugation formed between the biosensor and the biomolecule. Preferably, but not necessarily, the conjugate binding between the biosensor and the biomolecule is a covalent binding. However, the conjugate binding between the biosensor and the biomolecule may be selected such that the affinity between the biosensor and the biomolecule is sufficiently high compared to the affinity between the biosensor (second binding domain) and the ligand binding site of the biomolecule.

The term "ligand binding site" as used herein refers to a part of the biomolecule suitable for binding to a biomolecule specific ligand (e.g. small molecule or protein based biomarker or the like). Further, the ligand binding site binds to the second binding domain (competitive binding) of the biosensor of the invention.

Optionally, the ligand binding site of the biomolecule comprises an antigen binding site of an antibody.

Similar to the first aspect of the invention, in the second aspect of the invention, the second binding domain of the biosensor of the invention may be configured to bind with the biomolecule, i.e. to competitively bind to one of the binding sites of the biomolecule. Although the second binding domain may be any kind of binding domain having a certain affinity with one or more binding sites of the biomolecule, the use of a binding domain being an epitope is preferred.

In this second aspect of the invention, the invention further relates to an *in vitro* ligand-detecting method, comprising the steps of:

- contacting a sample with the biosensor of the second aspect of the invention;
- determining the change of the biosensor's luminescence in the presence of a sample; and
- determining the luminescence change in the presence of the sample to the quantitative and/or qualitative presence or absence of a ligand.

Optionally, the invention relates to a method wherein in the absence of a ligand the biomolecule binds to the second binding domain disrupting the interaction of the second luciferase domain with the first luciferase domain. In case the fluorophore is next to the second luciferase domain, in the biomolecule-bound state the fluorophore is not in close proximity to the complemented luciferase formed by the first luciferase domain and third luciferase domain, resulting in a low BRET between the complemented luciferase and the fluorophore.

Optionally, the invention relates to a method wherein, in the presence of a ligand the biosensor is in a ligand-bound state allowing that at least some of the first luciferase domain complements with the second luciferase domain. In case the fluorophore is next to the second luciferase domain, the BRET between the luciferase and the fluorophore increases in the ligand-bound state compared to the biosensor in the biomolecule-bound state.

In a third aspect of the invention, the invention relates to a kit-of-parts comprising the biosensor of the invention (both first and second aspects of the invention) wherein the biosensor comprises a first luciferase domain fused to a second luciferase domain via a semi-flexible linker containing two binding domains at the ends of the linker, and wherein the kit of parts further comprises a third luciferase domain.

Optionally, the kit-of-parts is in the form of a solution comprising at least the biosensor and the third luciferase domain. Further optionally, the concentration of the third luciferase domain is significantly higher compared to the concentration of the biosensor present in the solution.

Optionally, the kit-of-parts comprises an inert material with the biosensor and the third luciferase domain attached thereto. The inert material may be any kind of material suitable for the intended purpose of detecting biomolecules or ligands in solution. The configuration of the attachment of the biosensor of the invention and the third luciferase domain may be such that the third luciferase domain is in close proximity to the binding site of the first luciferase domain of the biosensor.

Detailed description The invention is defined in the appended claims.

Within this invention, we present a new approach that allows one-step detection of biomolecules (or analytical targets, such as antibodies) directly in solution using intramolecular complementation of split luciferase. The sensor design is highly modular, including a large luciferase fragment fused to two small luciferase fragments. One small luciferase fragment is fused to one terminus of the large luciferase fragment via a flexible GGS repeats linker. Another small luciferase fragment is fused to the other terminus of the large luciferase fragment via a semi-flexible linker containing two antibody binding epitopes at the ends of the linker. This protein switch can exist in two conformations, in which either the N- or the C-terminal small fragment binds to the large split luciferase domain and complements luciferase activity. A fluorescent acceptor dye (also referred to as 'fluorophore') is conjugated next to one of the two small luciferase domains, resulting in efficient BRET in only one of the two conformations. Bivalent binding of an antibody to two epitope sequences flanking the semi-flexible linker disrupts the interaction of the fluorescently-labeled small luciferase domains, which allows complementation of luciferase activity by the non-fluorescently-labeled small luciferase fragment, resulting in a decrease in BRET between luciferase and the fluorophore that can be monitored using simple luminescent read outs. Using the anti-HIV1 p17 antibody as an exemplary target, the intramolecular affinity of the split luciferase fragments was optimized to yield a bioluminescent sensor whose dynamic range reached 493% in the presence of pM concentrations of the target antibody ($K_d$=10 pM). A sensor that targets a completely different antibody can be obtained by simply replacing the epitope sequence, with the possibility to further customize this sensor's performance by tuning the intramolecular affinities of the split parts.

The invention relates to a detection method where a biosensor is used for detecting biomolecules (e.g. antibodies, antigens, proteins, etc.). In particular, this invention relates to a detection method where a biosensor is used for detecting antibodies. The biosensor is a large split luciferase fragment linked to two small split luciferase fragments via a flexible GGS repeats linker and a semi-flexible linker having two epitopes at the ends of the linker.

It is noted that the exact length and content of these linkers depend on the conformation and configuration of the biosensor as such. Both the semi-flexible linker and flexible linker should be long enough and flexible enough to allow intramolecular complementation. Hereto the flexibility of the linkers may be varied to further optimize biosensor specific systems.

Here an *in vitro* antibody-detecting method is provided. The method entails contacting a sample with a biosensor. The biosensor includes one large luciferase fragment, two small luciferase fragments, at least one fluorescent acceptor dye, a flexible GGS repeats linker and a semi-flexible linker containing two epitopes with affinity for the antibody. The method further entails determining the change of the biosensor's luminescence in the presence of a sample, and attributing the luminescence change in the presence of the sample to the quantitative or qualitative presence or absence of an antibody. In the absence of the antibody, the biosensor is in an antibody free state (closed state) that at least some of the large split luciferase fragment complements with small split luciferase fragment connected via the semi-flexible linker containing two epitopes and the fluorophore is spaced close (in close proximity) to the complemented luciferase. In different words, there is relatively high BRET between the luciferase and the fluorophore and the biosensor shows relatively high luminescence emission ratio of fluorophore (e.g. red light) to luciferase (e.g. blue light). A bivalent binding between two antigen binding domains present in the antibody and the two epitopes present at the ends of the semi-flexible linker changes the equilibrium between the antibody-free state and an antibody-bound state (open state) of the biosensor such that the BRET between the luciferase and the fluorophore is decreased and the biosensor shows relatively low luminescence emission ratio of red to blue light.

Also described is an *in vitro* antibody-detecting method. In this method, a sample is contacted with a biosensor which is displaceable between an antibody-free (closed) state and an antibody-bound (open) state. The biosensor includes three split luciferase (for example, but not limited to NanoLuc) fragments, two epitopes which have affinity for the antibody, at least one fluorophore (for example, but not limited to Cy3), a semi-flexible linker separating the two epitopes and a flexible GGS repeats linker. This method further entails determining the luminescence of the luciferase and fluorophore in the presence of the sample, and attributing the luminescence of the luciferase and fluorophore in the presence of the sample to the quantitative or qualitative presence or absence of the antibody. In the antibody-free (closed) state, there is high BRET between the complemented luciferase and fluorophore and thus a high luminescence emission ratio of fluorophore (e.g. red light) to luciferase (e.g. blue light). The binding of an antibody to the two epitopes changes the equilibrium between the antibody-free state and the antibody-bound state of the biosensor, which thereby drives the biosensor from the antibody-free state state to the antibody-bound state, such that the BRET between the complemented luciferase and fluorophore is decreased and a low luminescence emission ratio of red to blue light is observed.

Also described is a biosensor displaceable between an biomolecule-free (closed) state and an biomolecule-bound (open) state. The biosensor includes a large split luciferase fragment, two small split luciferase fragments, at least one fluorophore, two epitopes, a semi-flexible linker separating the two epitopes and at a flexible GGS repeats linker.

The term "displaceable" as used herein refers to the suitability of the biosensor to have different conformations (states). The displaceability of the biosensor relates to the freedom of the small split luciferase fragments to bind to the large split luciferase fragment or to be positioned at a certain distance from the binding site of the large split luciferase fragment, wherein the distance is determined by the linker (semi-flexible or flexible linker) fused to the respective small split luciferase fragment.

Optionally, in the absence of the antibody, the biosensor is in the closed state. Further optionally, in the absence of an antibody, the biosensor is in the closed state whereby at least some of large luciferase fragment complements with the small luciferase fragment connected via the linker containing two epitopes and the fluorophore is spaced close to the complemented luciferase. Still further optionally, in the closed state, the fluorophore is spaced close to the complemented luciferase, such that there is high BRET between the complemented luciferase and fluorophore and a high luminescence emission ratio of fluorophore (e.g. red light) to luciferase (e.g. blue light).

Optionally, binding of an antibody to the two epitopes changes the equilibrium between the closed (antibody free) and open (antibody bound) state of the biosensor, which thereby drives the biosensor from the closed state to the open state, such that the BRET between the complemented luciferase and fluorophore is decreased and a low luminescence emission ratio of fluorophore (e.g. red light) to luciferase (e.g. blue light) is observed.

Optionally, in the presence of an antibody, the biosensor is in the open state. Further optionally, in the presence of an antibody, a bivalent binding between two antigen binding domains present in said antibody and the two epitopes present at the ends of the linker in the biosensor drives the biosensor to the open state. Still further optionally, in the open state, the fluorophore is spaced apart from the complemented luciferase, thereby resulting in the decrease of the BRET between the complemented luciferase and fluorophore and a luminescence emission ratio of fluorophore (e.g. red light) to luciferase (e.g. blue light).

Optionally, luminescence emission ratio of fluorophore (e.g. red light) to luciferase (e.g. blue light) is proportional to the quantitative or qualitative presence or absence of the antibody.

Optionally, the large and small split luciferase fragments are protein domains or polypeptides capable of self-assembly into a bioluminescent complex. Optionally, the split luciferase fragments are split NanoLuc fragments.

Optionally, the large split luciferase fragment is selected from an amino acid sequence of SEQ ID NO: 19, or having an identity of at least 70%, 75%, 80%, 85%, 90%, 95%, 96 % , 97 %, 98 %, or 99 % to the amino acid sequence of SEQ ID NO: 19.

Optionally, each of the small split luciferase fragments are selected from the group of amino acid sequences consisting of SEQ ID NO: 1, SEQ ID NO: 2 or SEQ ID NO: 3, or having an identity of at least 70%, 75%, 80%, 85%, 90%, 95%, 96 % , 97 %, 98 %, or 99 % to the amino acid sequences of SEQ ID NO: 1, SEQ ID NO: 2 or SEQ ID NO: 3.

In an example, the affinity of the two small split luciferase fragment to the large split luciferase fragment differs. Preferably, the small split luciferase fragment fused to the large split luciferase fragment via the semi-flexible linker flanked by the two epitopes has a higher affinity compared to the affinity of the small split luciferase fragment fused to the large split luciferase fragment via the flexible GGS repeats linker.

Optionally, the affinity of the small split luciferase fragment fused to the large split luciferase fragment via the semi-flexible linker flanked by the two epitopes has an affinity which is at least 5 fold higher, preferably between 10 and 1500 fold higher, between 10 and 1000 fold higher, between 10 and 100 fold higher or between 10 and 50 fold higher compared to the affinity of the small split luciferase fragment fused to the large split luciferase fragment via the flexible GGS repeats linker.

Optionally, the two epitopes are polypeptides or polypeptide fragments having affinity for the antibody and capable of binding, optionally selectively binding, to the antibody, optionally to the antigen-binding fragments of the antibody.

Optionally, the semi-flexible linker is a polypeptide or polypeptide fragment including at least one flexible block of $(GSG)_6$. Further optionally, the linker further includes at least one $\alpha$-helical block. Still further optionally, the linker further includes at least one $\alpha$-helical block having six EAAAK repeats. Still further optionally, the linker further includes two $\alpha$-helical blocks, each having six EAAAK repeats. In general it is noted that the semi-flexible linker is chosen such that the linker has enough flexibility to change the state of the biosensor, i.e. from an biomolecule target-free state wherein the small split luciferase fragment fused to the semi-flexible linker is bound to the large split luciferase fragment, to an biomolecule-bound state wherein the epitopes flanking the semi-flexible linker are bound to the binding site(s) of the biomolecule (e.g. antigen-binding site of an antibody).

Optionally, each epitope is located at each respective end of the semi-flexible linker. Further optionally, the large split luciferase fragment and one of the small split luciferase fragment is located at each respective end of the linker. Still further optimally, a first epitope and the large split luciferase fragment are located at a first end of the linker, and a second epitope and the small split luciferase fragment are located at a second, opposing end of the linker.

Optionally, the flexible linker is a polypeptide or polypeptide fragment including at least ten GGS repeats. However, it is noted that the number of GGS repeats may vary. Even the content of the linker may vary and is not necessarily restricted to the use of GGS repeats. The content and length of the linker depends on the configuration of the large split luciferase fragment and position of linking of the small split luciferase fragment fused to the flexible linker, to the large split luciferase fragment (depending on which terminus of the large split luciferase fragment is used to link the flexible linker to).

Optionally, the fluorophore is a fluorescent chemical compound that can emit red light upon light excitation. Further optionally, the fluorophore is Cy3.

Optionally, the fluorophore is conjugated to the biosensor. Further optionally, the fluorophore is conjugated to the biosensor adjacent to the small split luciferase fragment connecting via the semi-flexible linker. Further optionally, the fluorophore is conjugated to the biosensor between the semi-flexible linker and the small split luciferase fragment. Alternatively, the fluorophore is conjugated to the biosensor adjacent to the small split luciferase fragment connecting via the flexible linker. In such alternative configuration, it is noted that in an antibody-free state the BRET between the complemented luciferase and fluorophore is decreased and a low luminescence emission ratio of fluorophore (e.g. red light) to luciferase (e.g. blue light) is observed, whereas in an antibody-bound state the fluorophore next to the small split luciferase fragment connecting via the flexible linker is in close proximity of the complemented luciferase resulting in an increased BRET between the complemented luciferase and fluorophore.

In an example, the fluorophore is incorporated in the biosensor via a maleimide coupling to the free thiol group of the cysteine in the biosensor. Optionally, the fluorophore is incorporated in the biosensor between the small split luciferase fragment and the semi-flexible linker via a maleimide coupling to the free thiol group of the cysteine in the biosensor. In other words, the position of the fluorophore in the biosensor, i.e. before (between the semi-flexible linker and the small split luciferase fragment) and/or after the small split luciferase fragment is controlled by the introduction of the cysteine in the amino acid sequence of the biosensor.

In another example, the fluorophore is incorporated in the biosensor via an alkyne coupling to the azide group of the non-canonical amino acid para-azidophenylalanine (pAzF) in the biosensor. Optionally, the fluorophore is incorporated in the biosensor between the small split luciferase fragment and the semi-flexible linker via an alkyne coupling to the azide group of the para-azidophenylalanine in the biosensor. Optionally, the para-azidophenylalanine was bioorthogonally incorporated into the biosensor at the desired positions using the amber stop codon (TAG). In other words, the position of the fluorophore in the biosensor, i.e. before (between the semi-flexible linker and the small split luciferase fragment) and/or after the small split luciferase fragment is controlled by the introduction of the amber stop codon (TAG) codon in the amino acid sequence of the biosensor.

In addition to the detection of antibodies, the biosensor of the current invention as well as the detection method of the current invention can be equally applied to the detection of other biomolecules. For example, using the sensor in a competitive fashion allows the detection of antigens, small molecules and other biomarkers (e.g. c-reactive protein).

Also within this invention, we present a new approach that allows one-step detection of small molecules, protein based biomarkers or the like directly in solution using intramolecular complementation of split luciferase wherein the biosensor is conjugated to a biomolecule (e.g. antibody, antigen, protein, aptamer, etc.) wherein the biomolecule comprises a binding site for binding a ligand (e.g. small molecule, protein based biomarker or the like). The biosensor is highly modular, including a linker, e.g. the semi-flexible linker of the present invention, comprising a first end and a second end, wherein the first end of the linker is fused to one terminus of a first luciferase domain, such as the large (split) luciferase domain of the present invention, via a first binding domain, and wherein the second end of the linker is fused to a second luciferase domain, such as the small (split) luciferase domain of the present invention, via a second binding domain. The biosensor might optionally comprise a third luciferase domain, such as the small (split) luciferase domain of the present invention, fused to the other terminus of the first luciferase domain via a spacer, e.g. the flexible GGS repeats linker of the present invention. The first binding domain is configured to form a conjugated system with the biomolecule (e.g. antibody), whereas the second binding domain is configured to bind to the ligand binding site of the biomolecule (e.g. the antigen binding site of an antibody). The biosensor exists in two conformations, in which either the second luciferase domain or, in case present, the third luciferase domain binds to the first luciferase domain to form a complemented luciferase and complements luciferase activity. Further, a fluorescent acceptor dye (fluorophore) is conjugated next to one of the second or third luciferase domains, e.g. one of the two small luciferase domains of the present invention resulting in efficient Bioluminescence Resonance Energy Transfer (BRET) between the luciferase and the fluorophore in only one of the two conformations.

In the absence of a ligand (e.g. small molecule or protein based biomarker), the biomolecule is conjugated to the first binding domain and the ligand binding site of the biomolecule is bound to the second binding domain disrupting the interaction of the second luciferase domain with the first luciferase domain and therefore allows interaction of the third luciferase domain, in case present, with the first luciferase domain. In case the second luciferase domain is the fluorescently-labeled luciferase domain, the complementation of luciferase activity by the non-fluorescently-labeled third luciferase domain results in a decrease in BRET between the complemented luciferase and the fluorophore that can be monitored using simple luminescent read outs. Alternatively, in case the third luciferase domain is the fluorescently-labeled luciferase domain, the complementation of luciferase activity by the fluorescently-labeled third luciferase domain results in an increase in BRET between the complemented luciferase and the fluorophore that can be monitored using simple luminescent read outs.

In the presence of a ligand, the binding of the biomolecule (e.g. antibody) to the second binding domain of the biosensor is disrupted, allowing the second luciferase domain to interact with the first luciferase domain. In case the second binding domain is the fluorescently-labeled luciferase domain, the complementation of luciferase activity by the fluorescently-labeled second luciferase domain results in an increase in BRET between the luciferase and the fluorophore that can be monitored using simple luminescent read outs.

In an embodiment of the invention, an antibody conjugated biosensor is provided. The antibody conjugated biosensor including a linker, e.g. the semi-flexible linker of the present invention, comprising a first end and a second end, wherein the first end of the linker is fused to one terminus of a first luciferase domain, such as the large (split) luciferase domain of the present invention, via an antibody conjugating binding domain, and wherein the second end of the linker is fused to a second luciferase domain, such as the small (split) luciferase domain of the present invention, via an antibody binding epitope. The biosensor might optionally comprise a third luciferase domain, such as the small (split) luciferase domain of the present invention, fused to the other terminus of the first luciferase domain via a spacer, e.g. the flexible GGS repeats linker of the present invention. The antibody conjugating binding domain is configured to form a conjugated system with an antibody, whereas the antibody binding epitope is configured to bind to the binding site of the antibody (e.g. one of the paratopes of the antibody). The biosensor exists in two conformations, in which either the second luciferase domain or the third luciferase domain (either linked to the first luciferase domain or present as a component in, for example, a solution comprising the antibody conjugated biosensor) binds to the first luciferase domain to form a complemented luciferase and complements luciferase activity. Further, a fluorescent acceptor dye (fluorophore) is conjugated next to one of the second or third luciferase domains, e.g. one of the two small luciferase domains of the present invention resulting in efficient Bioluminescence Resonance Energy Transfer (BRET) between the luciferase and the fluorophore in only one of the two conformations. Preferably, the fluorophore is conjugated next to the second luciferase domain.

Optionally, the first binding domain or the antibody conjugating binding domain comprises antibody Fc-region affinity ligands. The antibody Fc-region affinity ligands may be selected from the group consisting of (natural) immunoglobulin binding proteins, immunoglobulin-derived products, such as engineered versions of immunoglobulin binding proteins, artificial binding proteins, (short) peptides, aptamers and synthetic small-molecular-weight compounds. Examples of such antibody Fc-region affinity ligands are for example described by Kruljec, et al. (Bioconjugate Chem. 2017, 28, 2009). Optionally, the first binding domain or the antibody conjugating binding domain is an immunoglobulin-binding protein. Further optionally, the first binding domain or the antibody conjugating binding domain is a protein G derived domain having an amino acid sequence of SEQ ID NO: 16, or having an identity of at least 70%, 75%, 80%, 85%, 90%, 95%, 96 % , 97 %, 98 %, or 99 % to the amino acid sequence of SEQ ID NO: 16.

The term "antibody Fc-region affinity ligands" as used herein refers to ligands having affinity to at least the Fc-region binding site of an antibody. Preferably, the antibody Fc-region affinity ligands of the invention having a higher affinity to the Fc-region binding site of an antibody compared to the affinity of the ligands to another binding site, e.g. Fab-region binding site, of that same antibody.

Optionally, the second binding domain is configured to provide a competition between the intermolecular binding of the second binding domain and the ligand (e.g. small molecule or protein based biomarker) to an antigen-binding site of a ligand specific antibody. Therefore, the second binding domain may be selected from the group consisting of antibody antigen-binding site ligands including epitopes, meditopes, mimotopes, artificial binding proteins, (short) peptides, low-molecular-weight analogs or the like. Preferably, the second binding domain comprises an antibody binding epitope or antibody binding low-molecular-weight analog.

The term "antibody antigen-binding site ligands" as used herein refers to ligands having affinity to at least the antigen-binding site of an antibody. Preferably, the antibody antigen-binding site ligands of the invention having a higher affinity to the antigen-binding site of an antibody compared to the affinity of the ligands to another binding site, e.g. Fab-region or Fc-region binding sites, of that same antibody. In order to provide competition between the antibody antigen-binding site ligands of the invention and the analyte of interests (e.g. small molecule or protein based biomarker), the binding of the ligands to the antigen-binding site of an antibody is reversible.

Optionally, the second binding domain is an antibody binding epitope. For example, the antibody binding epitope may be selected from the group of amino acid sequences consisting of SEQ ID NO: 4, SEQ ID NO: 5, SEQ ID NO: 17, SEQ ID NO: 18, SEQ ID NO: 23 and SEQ ID NO: 24, or having an identity of at least 70%, 75%, 80%, 85%, 90%, 95%, 96 % , 97 %, 98 %, or 99 % to the amino acid sequence of SEQ ID NO: 4, SEQ ID NO: 5, SEQ ID NO: 17, SEQ ID NO: 18, SEQ ID NO: 23 or SEQ ID NO: 24.

Optionally, the fluorophore is a fluorescent chemical compound that can emit red light upon light excitation. Further optionally, the fluorophore is Cy3.

Optionally, the fluorophore is conjugated to the biosensor. Further optionally, the fluorophore is conjugated to the biosensor adjacent to the second luciferase domain. Further optionally, the fluorophore is conjugated to the biosensor between the linker and the second luciferase domain.

In an embodiment of the invention, the fluorophore is incorporated in the biosensor via a maleimide coupling to the free thiol group of the cysteine in the biosensor. Optionally, the fluorophore is incorporated in the biosensor between the second luciferase domain and the linker via a maleimide coupling to the free thiol group of the cysteine in the biosensor. In other words, the position of the fluorophore in the biosensor, i.e. before (between the linker and the second luciferase domain) and/or after the second luciferase domain is controlled by the introduction of the cysteine in the amino acid sequence of the biosensor.

In another embodiment of the invention, the fluorophore is incorporated in the biosensor via an alkyne coupling to the azide group of the non-canonical amino acid para-azidophenylalanine (pAzF) in the biosensor. Optionally, the fluorophore is incorporated in the biosensor between the second luciferase domain and the linker via an alkyne coupling to the azide group of the para-azidophenylalanine in the biosensor. Optionally, the para-azidophenylalanine was bioorthogonally incorporated into the biosensor at the desired positions using the amber stop codon (TAG). In other words, the position of the fluorophore in the biosensor, i.e. before (between the linker and the second binding domain) and/or after the second binding domain is controlled by the introduction of the amber stop codon (TAG codon) in the amino acid sequence of the biosensor.

Optionally, the fluorophore is part of the second binding domain. In particular in case the second binding domain comprises a small molecule analog for binding the antigen-binding site of the antibody conjugated to the first binding domain of the biosensor, the small molecule analog may be fused to the linker via the fluorophore.

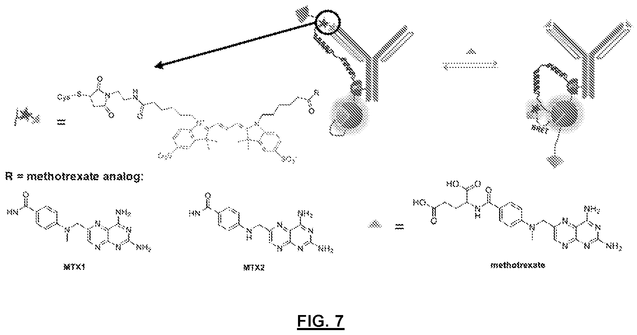

For example, the second binding domain may comprise a small molecule analog selected from the group consisting of methotrexate analogs to detect the chemotherapy drug methotrexate (MTX). Preferably, the fluorophore is used to conjugate the methotrexate analog to the cysteine in the biosensor via the fluorophore (Figure 7).

Exemplary embodiments of the invention

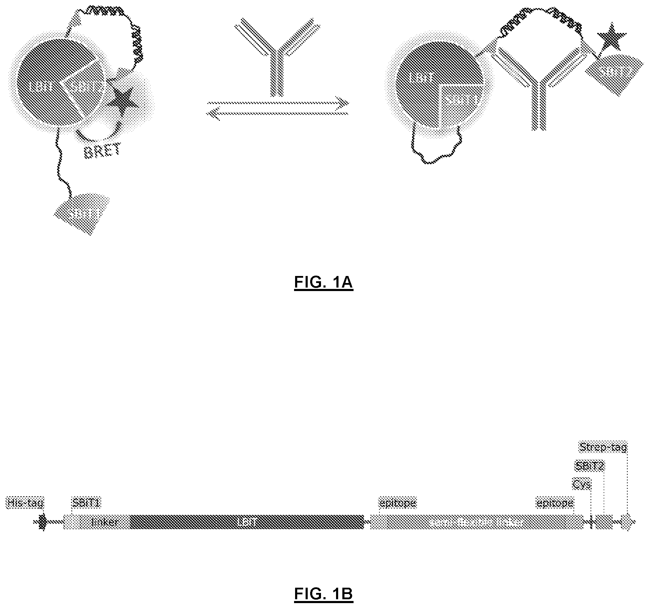

The biosensor can contain one large split luciferase fragment (LBiT), two small split luciferase fragments (SBiT1 and SBiT2) and a synthetic fluorophore. The LBiT is connected to SBiT2 via a long semi-flexible linker and the fluorophore is introduced adjacent to the SBiT2, forming a complemented bioluminescent complex in the absence of its target antibody with efficient BRET between the bioluminescent complex and the fluorophore (Figure 1). Peptide epitopes specific to the antibody of interest are included in the semi-flexible linker, one is next to the LBiT and the other adjacent to the SBiT2. The other terminus of LBiT is fused to SBiT1 via a short flexible linker. Binding of a single antibody to both epitopes separates the complemented bioluminescent complex and pushes away the fluorophore. Meanwhile, the LBiT assembles with the SBiT1 tethered via the short flexible linker and forms the bioluminescent complex. This results in a decrease of the BRET efficiency between the bioluminescent complex and fluorophore.

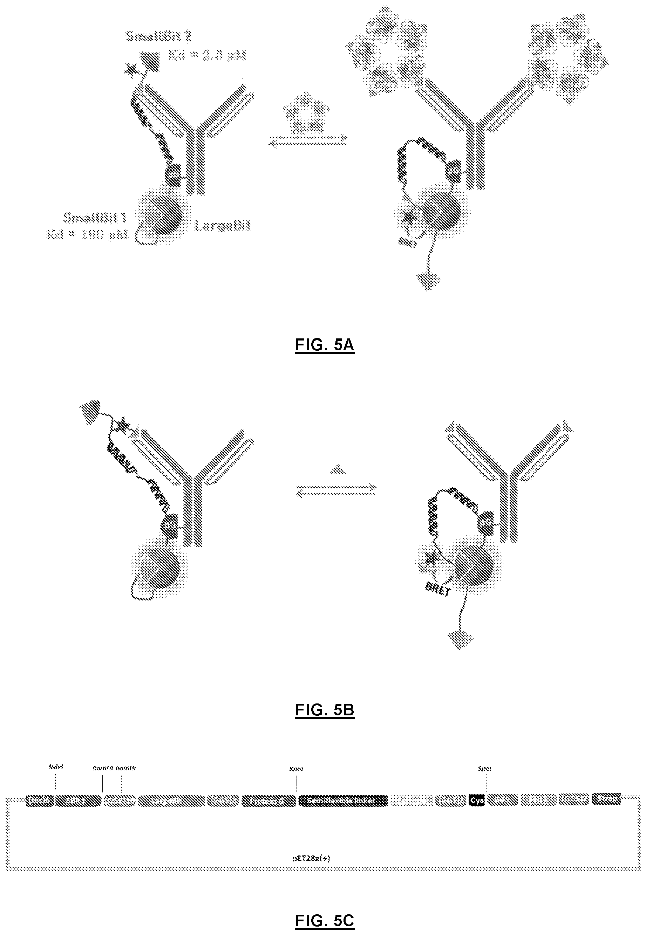

Alternatively, the biosensor can be conjugated to an Fc-region of an antibody through protein G included in the semi-flexible linker next to the LargeBit (LBiT) (Figure 5). The antigen-binding site of the antibody conjugated to the biosensor through protein G binds to a peptide epitope (Figure 5A) or small molecule analog (Figure 5B) included in the semi-flexible linker adjacent to the SmallBit 2 (SBiT2). Binding of an analyte of interest (e.g. small molecule or protein biomarker) to the antigen-binding site of the antibody conjugated to the biosensor allows the SmallBit 2 (SBiT2) to form the complemented bioluminescent complex bringing the fluorophore in close proximity of the LargeBit (LBiT). This results in an increase of the BRET efficiency between the bioluminescent complex and fluorophore.

The feasibility of the technology of this invention was demonstrated using split NanoLuc. The affinity between LBiT and SBiT was modulated to yield single-protein bioluminescent sensors that allow detection of pM concentrations of specific antibodies with large signal change or mM concentrations of small molecules or protein biomarkers with large signal change. Moreover, the modular architecture of these sensors allows changing of target specificity by simple exchange of epitope sequences and systematic tuning of the sensor response by tuning the relative affinities of the SBiT fragments.

Description of the figures

Figure 1A shows an antibody sensor based on the antibody-induced disruption of an intramolecular complex between split luciferase fragments which results in a change of BRET efficiency. The sensor is labeled with a fluorophore (star). Binding of an antibody to the epitopes in the semi-flexible linker pulls the fluorophore and luciferase apart, changing the color of emission from red to blue.
Figure 1B shows a schematic structure of the sensor sequence of NB-LUMABS-1
Figure 2A shows luminescence spectra of 1 pM sensor in the absence (red line; •) and presence (blue line; ■) of 1.25 nM anti-HIV1-p17 antibody. (B) Antibody titration at a sensor concentration of 1 pM. Error bars represent mean ± SD (n=3).
Figure 2B shows antibody titration at a sensor concentration of 1 pM. Error bars represent mean ± SD (n=3).
Figure 3 shows the characterization of NB-LUMABS-2, -3, -4 (Figure 3A), -5, -6, -7 (Figure 3B) including the luminescence spectra of 1 pM sensor in the absence (red line; •) and presence (blue line; ■) of 1.25 nM anti HIV1-p17 antibody and the antibody titration at a sensor concentration of 1 pM. Error bars represent mean ± SD (n=2).
Figure 4 shows the characterization of TRAS-NB-LUMSABS-1, -2 and -3 including the luminescence spectra of 100 pM sensor in the absence (red line; •) and presence (blue line; ■) of 1 μM trastuzumab and the antibody titration at a sensor concentration of 100 pM. Error bars represent mean ± SD (n=3).
Figure 5 shows pG-NB-LUMABS sensors allowing ratiometric bioluminescent detection of in principle any ligand. Figure 5A shows the detection of CRP as an example of a protein biomarker. Figure 5B shows the detection of a small molecule. Detection is based on competition between intermolecular binding of a peptide epitope (Figure 5A) or small-molecule analogue (Figure 5B) and the ligand to a ligand-specific biomolecule (e.g. monoclonal antibody). Figure 5C shows a schematic overview of the pG-NB-LUMABS pET28a(+) vector, with a N-terminal His-Tag and a C-terminal Streptavidin tag for purification.
Figure 6 shows the characterization of CTX-NB-LUMABS variants. Luminescence spectra of 100 pM sensor in the absence (red line; •) and presence (blue line; ■) of 8.3 μM cetuximab. Antibody titration to 100 pM of sensor in PBS buffer (pH7.4, 1 mg/ml BSA). Error bars represent mean ± SEM (n = 3). The red lines (•) represent fits to equation 1.
Figure 7 shows a MTX-pG-NB-LUMABS for the detection of the small molecule methotrexate.

Figure 8 shows the luminescence spectra of 2 nM pG-NB-LUMABS-17 in the presence and absence of 0.8 mM unpurified peptide 17 (Figure 8A) and the emission ratio of 2 nM pG-NB-LUMABS-epitope 17 upon increasing concentration of peptide 17.

Sensor design

Figure 1 shows the general design of the sensor. In this example, split NanoLuc was chosen as a complementation reporter because the complemented complex is blue luminescent and a variety of SBiT variants are available with affinities between 0.7 nM and 190 $\mu$M toward LBiT (Dixon, et al. ACS Chem. Biol., 2016, 11, 400; SEQ ID NO: 19).

In an exemplary design we focused on developing a sensor for the detection of the anti-HIV1-p17 antibody. Several well-characterized linear epitope sequences are available for this antibody, which has made it a popular choice for the development of new homogeneous antibody detection assays. The linker between the LBiT and SBiT1 has flexible blocks of $(GGS)_{10}$. The linker between the LBiT and SBiT2 initially has two epitopes (SEQ ID NO: 4, $K_d$=42 nM) specific for the HIV1-p17-antibody that are separated by three flexible blocks of $(GSG)_6$ and two $\alpha$-helical blocks each having six EAAAK repeats (SEQ ID NO: 20). The linker was also used in some reported sensor protein based on the same switching strategy (see: Golynskiy, et al. ChemBioChem, 2010, 11, 2264; Banala, et al. ACS Chem. Biol., 2013, 8, 212; Arts, et al. Anal. Chem., 2016, 88, 4525; Van Rosmalen, et al. Anal. Chem., 2018, DOI: 10.1021/acs.analchem.8b00041; and Arts, et al. ACS Sens., 2017, 2, 1730). One cysteine residue was included adjacent to SBiT2 for fluorophore conjugation.

One variant (NB-LUMABS-1; SEQ ID NO: 6) was constructed in an exemplary embodiment containing SBiT1 with $K_d$ of 190 $\mu$M (SEQ ID NO: 1) and SBiT2 with $K_d$ of 2.5 $\mu$M (SEQ ID NO: 2), and a cysteine residue before the N-terminus of SBiT2. The sensor protein was expressed in *E. coli* BL21 (DE3) and purified using an N-terminal His-tag and a C-terminal Strep-tag. This two-step purification protocol ensures the isolation of full length protein only, without truncated version of the sensor lacking e.g. SBiT domain. The sensor protein was conjugated with synthetic fluorophore Cy3 via thiol-maleimide reaction.

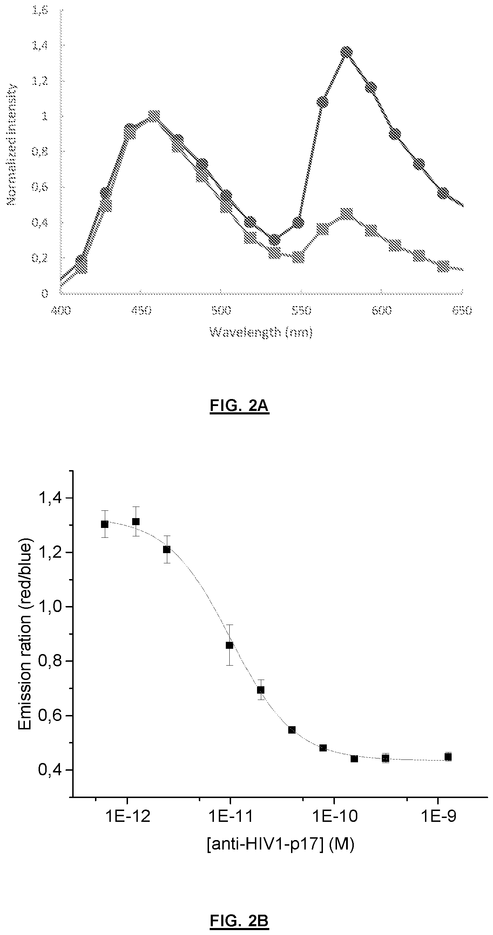

Bioluminescent scan of NB-LUMABS-1 showed a higher Cy3 emission peak at 563nm than the NanoLuc emission at 460nm in the absence of anti-HIV1-p17 antibody, with an emission ratio of 1.3 which indicates very efficient BRET between NB and Cy3. Addition of anti-HIV1-p17 antibody resulted in a significant decrease of the Cy3 emission peak, indicating a large decrease of BRET efficiency. Titration experiments yielded apparent dissociation constants ($K_{d,app}$) of 10.0 $\pm$ 0.5 pM and a large dynamic range of 218% (Figure 2).

Tuning sensor performance

To establish the influence of cysteine residue position, two variants were constructed containing either one cysteine residue after the C-terminus of SBiT2 (NB-LUMABS-2; SEQ ID NO: 7) or two cysteine residues both before the N-terminus of SBiT2 and after the C-terminus of SBiT2 (NB-LUMABS-3; SEQ ID NO: 8). NB-LUMABS-2 showed a relatively low emission ratio (red to blue) in the absence of anti-HIV1-p17 antibody. It suggests that Cy3 is farther away from the substrate binding site of luciferase. NB-LUMABS-3 also showed a low relatively low emission ratio (red to blue) at the antibody-free state. We assume that the conjugation with two Cy3 group might affect the assembly of SBiT2 with LBiT.

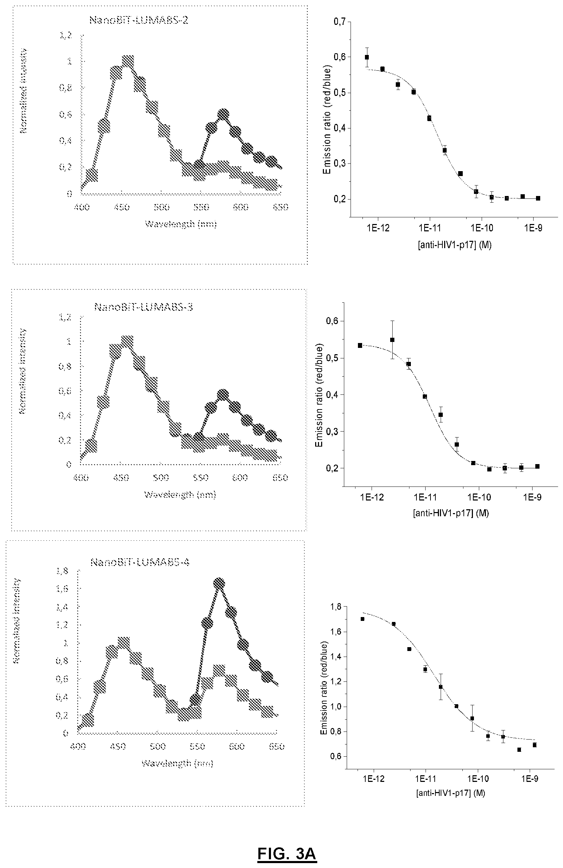

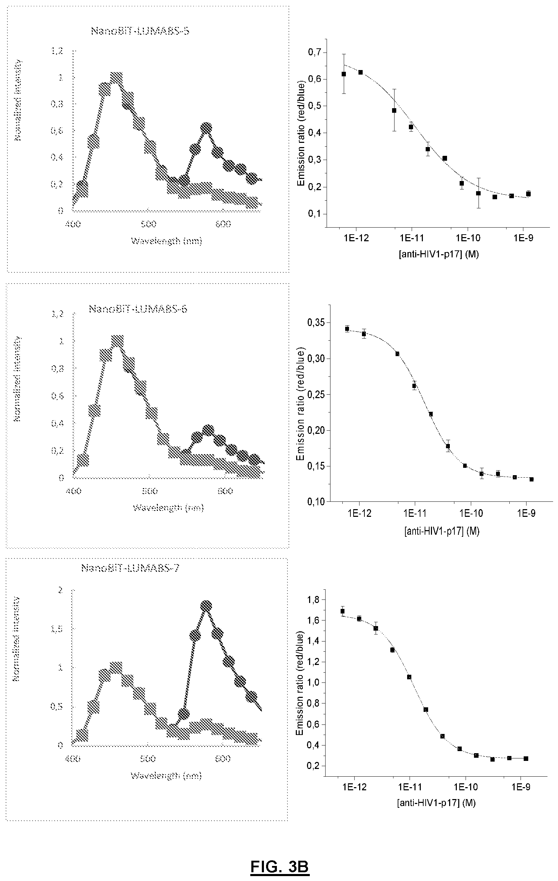

To establish the influence of LBiT-SBiT affinity, four variants were constructed containing different combination of SBiTs. NB-LUMABS-4 (SEQ ID NO: 9) contained SBiT1 with $K_d$ of 190 $\mu$M (SEQ ID NO: 1) and SBiT2 with $K_d$ of 0.18 $\mu$M (SEQ ID NO: 3). NB-LUMABS-5 (SEQ ID NO: 10) contained two same SBiTs with $K_d$ of 190 $\mu$M (SEQ ID NO: 1). NB-LUMABS-6 (SEQ ID NO: 11) contained two same SBiTs with $K_d$ of 2.5 $\mu$M (SEQ ID NO: 2). NB-LUMABS-7 (SEQ ID NO: 12) contained SBiT1 with $K_d$ of 2.5 $\mu$M (SEQ ID NO: 2) and SBiT2 with $K_d$ of 0.18 $\mu$M (SEQ ID NO: 3). NB-LUMABS-4 showed a high emission ratio in the absence of antibody, but it moderately decreased by antibody biding. NB-LUMABS-5 and -6 containing two same SBiTs in a single sensor protein showed low emission ratio at antibody-free state, which indicates that in these sensors SBiT1 already complements NanoLuc activity in a significant fraction of the sensor proteins. For NB-LUMABS-7, the emission ratio reached 1.8 at antibody-free state and it significantly decreased upon addition of antibody. The dynamic range (DR) reached 493%. Antibody titration experiments yielded apparent affinities between 10 and 15 pM for these four sensor variants, which are similar to that obtained for the NB-LUMABS-1 (Figure 3).

Table 1. Properties of various NB-LUMABS (NB-LUMABS) variants targeting at anti-HIV1-p17 antibody.

| Sensor name | SBiT1 | SBiT2 | Cys position | DR | $K_{d,app}$ (pM) |
|---|---|---|---|---|---|
| NB-LUMABS-1 | $K_d$=190 $\mu$M | $K_d$=2.5 $\mu$M | before SBiT2 | 218% | 10.0 $\pm$ 0.5 |

(continued)

| Sensor name | SBiT1 | SBiT2 | Cys position | DR | $K_{d,app}$ (pM) |
|---|---|---|---|---|---|
| NB-LUMABS-2 | $K_d$=190 $\mu$M | $K_d$=2.5 $\mu$M | after SBiT2 | 177% | 13.7 $\pm$ 1.3 |
| NB-LUMABS-3 | $K_d$=190 $\mu$M | $K_d$=2.5 $\mu$M | Before and after SBiT2 | 182% | 12.1 $\pm$ 0.6 |
| NB-LUMABS-4 | $K_d$=190 $\mu$M | $K_d$=0.18 $\mu$M | before SBiT2 | 138% | 14.2 $\pm$ 4.7 |
| NB-LUMABS-5 | $K_d$=190 $\mu$M | $K_d$=190 $\mu$M | before SBiT2 | 252% | 11.7 $\pm$ 3.7 |
| NB-LUMABS-6 | $K_d$=2.5 $\mu$M | $K_d$=2.5 $\mu$M | before SBiT2 | 160% | 15.2 $\pm$ 1.0 |
| NB-LUMABS-7 | $K_d$=2.5 $\mu$M | $K_d$=0.18 $\mu$M | before SBiT2 | 493% | 11.8 $\pm$ 0.5 |

Tuning sensor selectivity

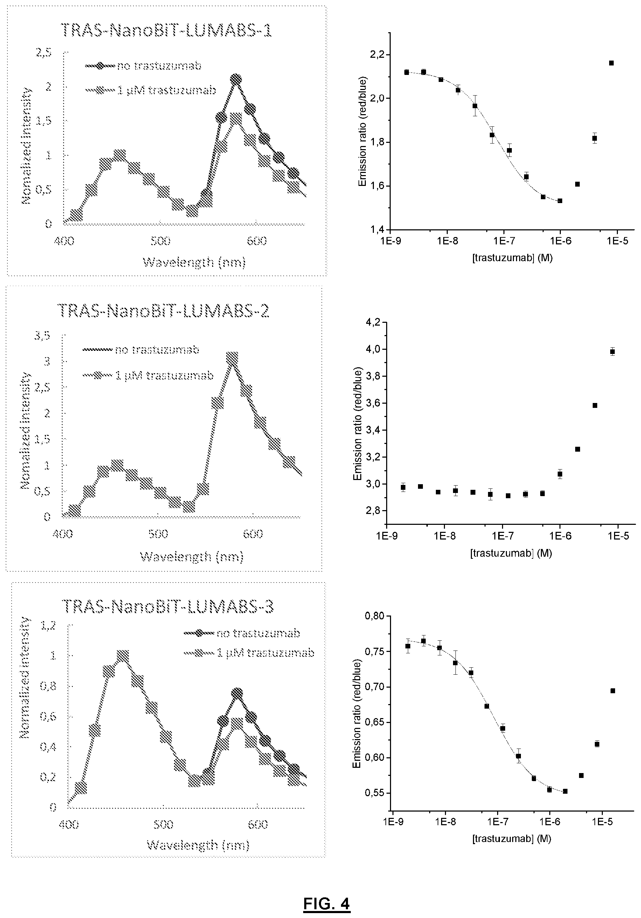

To challenge the modularity of our sensor design we tested whether the epitope sequences could be exchanged for epitope sequences targeting a different antibody. Sensors targeting therapeutic antibody trastuzumab (TRAS-NB-LUMABS-1, SEQ ID NO: 13; TRAS-NB-LUMABS-1, SEQ ID NO: 14; and TRAS-NB-LUMABS-3, SEQ ID NO: 15) were constructed by replacing the epitope sequences present in NB-LUMABS-1, -5 and -7 with a trastuzumab-binding mimitope QLGPYELWELS (SEQ ID NO: 5). Trastuzumab is used in the treatment of Her2-positive metastatic breast cancers. Population pharmacokinetics indicate interpatient variabilities in clearance and 10% patients with fast clearance, resulting in drug levels below the minimally effective concentration (Bruno, et al. Cancer Chemother. Pharmacol. 2005, 56, 361). The monovalent affinity of mimitope to trastuzumab was 294 nM. For these three sensors, efficient BRET was observed at the trastuzumab-free state (Figure 4). Addition of 1 $\mu$M trastuzumab to TRAS-NB-LUMABS-1 resulted in a moderate decrease in BRET, with a dynamic range of 37%. An increase of emission ratio was observed when adding large amount of trastuzumab. Possibly two molecules of antibody bind with one molecule of sensor, and thus the sensor restored as the closed state. TRAS-NB-LUMABS-2 kept in the closed state during antibody titration. We assume that the affinity between LBiT and SBiT2 is so high that the weak epitope affinity failed to lead to bivalent antibody binding. TRAS-NB-LUMABS-3 exhibited moderate change of emission ratio upon addition of trastuzumab, with a dynamic range of 36%. Titration of trastuzumab resulted in $K_{d,app}$ of 74.0 $\pm$ 9.4 nM for TRAS-NB-LUMABS-1 and 85.7 $\pm$ 6.3 nM for TRAS-NB-LUMABS-3.

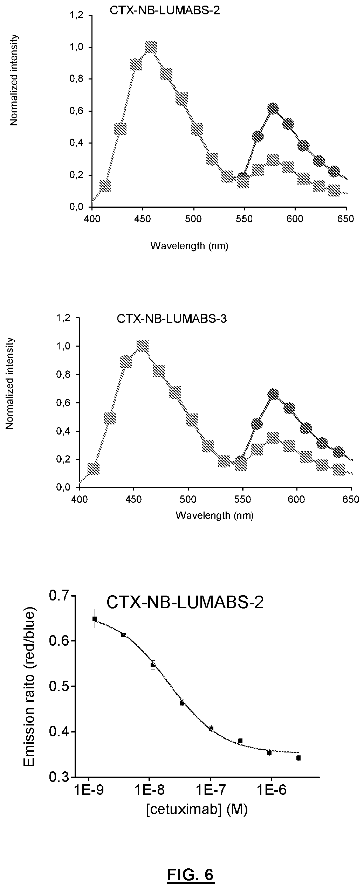

Additionally, sensors targeting therapeutic antibody cetuximab (CTX-NB-LUMABS-1, SEQ ID NO: 25; CTX-NB-LUMABS-2, SEQ ID NO: 26; and CTX-NB-LUMABS-3, SEQ ID NO: 27) were constructed by respectively replacing the epitope sequences present in NB-LUMABS-1 and -2 with a cetuximab-binding meditope CVFDLGTRRLRC (monovalent $K_d$ of 61 nM; SEQ ID NO: 23) and NB-LUMABS-1 with a cetuximab-binding meditope CQFDLSTRRLKC (monovalent $K_d$ of 270 nM; SEQ ID NO: 24). Cetuximab is a clinically important anticancer therapeutic antibody. Since cetuximab binds to a discontinuous conformational epitope on the cancer marker EGFR, no linear epitope sequences are available. Nevertheless, disulfide-linked cyclic meditope peptides were identified with sufficient affinity to cetuximab and were used to construct a blue-green emitting LUMABS sensor for cetuximab with a relatively modest change in emission ratio (DR ~60%) (see: Van Rosmalen, et al. Anal. Chem., 2018, 90, 3592). As the presence of cysteine residues in the meditope peptides precludes the use of cysteine-maleimide chemistry to introduce the Cy3 dye, we instead introduced the non-canonical amino acid para-azidophenylalanine (pAzF) to allow site-specific conjugation with a DBCO-functionalized fluorophore via strain-promoted azide-alkyne click chemistry (SPAAC). In order to incorporate pAzF, a TAG amber stop codon was introduced either before the N-terminus (CTX-NB-LUMABS-1; SEQ ID NO: 25) or after the C-terminus of SBiT2 (CTX-NB-LUMABS-2; SEQ ID NO: 26). Co-expression with the orthogonal tRNA synthetase/tRNA pair for pAzF allowed successful incorporation of pAzF into the cetuximab sensor variants at the desired position. CTX-NB-LUMABS-1 showed bright Cy3 emission in the absence of cetuximab and a significant decrease in emission ratio upon antibody binding (Figure 6). A dynamic range of 233 $\pm$ 12% was achieved, representing a 4-fold improvement compared to the blue-green CTX-LUMABS sensors (Van Rosmalen, et al. Anal. Chem. 2018, 90, 3592) while an overall $K_d$ of 34.7 $\pm$ 3.7 nM obtained from fitting the titration data was found to be similar (Figure 6). Introduction of Cy3 at the C-terminus of SBiT2 in CTX-NB-LUMABS-2 substantially decreased the BRET efficiency in the antibody-free state (Figure 6), which is consistent with the results obtained for HIV-NB-LUMABS-2. We also constructed a sensor variant (CTX-NB-LUMABS-3) containing the cyclic cetuximab meditope with a weaker affinity (SEQ ID NO: 24). Titration experiments with CTX-NB-LUMABS-3 showed a $K_d$ of 189 $\pm$ 16 nM, enabling this sensor to reliably measure high cetuximab concentrations (Figure 6). The lower affinity of CTX-NB-LUMABS-3 for cetuximab also resulted in a more rapid response compared to that of CTX-NB-LUMABS-1. These results demonstrate that the NB-LUMABS sensor format can be reconfigured to detect antibodies recognizing disulfide constrained cyclic peptides, yielding two cetuximab sensors whose combined responsive regime covers the clinically relevant cetuximab concentration range (25 nM - 2.3 $\mu$M).

These results further show that the framework developed for the exemplary anti-HIV1-p17 antibody can be used to easily develop sensor for other antibodies.

Table 2. Properties of various NB-LUMABS variants targeting at therapeutic antibodies.

| Sensor name | SBiT1 | SBiT2 | Fluorophore position | DR | $K_{d,app}$ (nM) |
|---|---|---|---|---|---|
| TRAS-NB-LUMABS-1 | $K_d$=190 μM | $K_d$=2.5 μM | before SBiT2 | 37% | 74.0 ± 9.4 |
| TRAS-NB-LUMABS-2 | $K_d$=2.5 μM | $K_d$=0.18 μM | before SBiT2 | 0% | - |
| TRAS-NB-LUMABS-3 | $K_d$=190 μM | $K_d$=190 μM | before SBiT2 | 36% | 85.7 ± 6.3 |
| CTX-NB-LUMABS-1 | $K_d$=190 μM | $K_d$=2.5 μM | before SBiT2 | 233% | 34.7 ± 3.7 |
| CTX-NB-LUMABS-2 | $K_d$=190 μM | $K_d$=2.5 μM | after SBiT2 | 110% | 20.7 ± 3.4 |
| CTX-NB-LUMABS-3 | $K_d$=190 μM | $K_d$=2.5 μM | before SBiT2 | 88% | 189 ± 16 |

Experiments

Cloning and mutagenesis

Synthetic DNA sequences encoding NB-LUMABS-1 (SEQ ID NO: 6) was ordered from GenScript (Piscataway, USA). NB-LUMABS-2 (SEQ ID NO: 7), NB-LUMABS-3 (SEQ ID NO: 10), NB-LUMABS-4 (SEQ ID NO: 9), NB-LUMABS-5 (SEQ ID NO: 10), NB-LUMABS-6 (SEQ ID NO: 11) and NB-LUMABS-7 (SEQ ID NO: 12) were constructed from NB-LUMABS-1 by site directed mutagenesis using specific primers (Table 3). The QuickChange site-directed mutagenesis kit (Agilent Technologies) was used in accordance with the manufacturer's instructions to introduce the mutations of interest.

To construct TRAS-NB-LUMABS-1 (SEQ ID NO: 13), TRAS-NB-LUMABS-2 (SEQ ID NO: 14) and TRAS-NB-LUMABS-3 (SEQ ID NO: 15), the gene encoding the semi-flexible liner including trastuzumab mimitope (QLGPYELWELSH) was cloned into the pET28a plasmids encoding NB-LUMABS-1, -5 or -7 via restriction-ligation approach using Kpnl and Spel. All cloning and mutagenesis results were confirmed by Sanger sequencing (StarSEQ GmbH).

To construct CTX-NB-LUMABS-1 (SEQ ID NO: 25), CTX-NB-LUMABS-2 (SEQ ID NO: 26) and CTX-NB-LUMABS-3 (SEQ ID NO: 27), pET28a(+) plamids encoding CTX-LUMABS which were prepared according to the method disclosed by Van Rosmalen, et al. (Anal. Chem. 2018, 90, 3592) were digested with Kpnl-HF and Spel-HF restriction enzymes. The linkers including cetuximab meditope, i.e. CTX-NB-LUMABS-1 and -2 with meditope CVFDLGTRRLRC (SEQ ID NO: 23), and CTX-NB-LUMABS-3 with meditope CQFDLSTRRLKC (SEQ ID NO: 24) were then cloned into the pET28a(+) plasmids encoding NB-LUMABS-1 (SEQ ID NO: 6) by restriction-ligation approach with Kpnl-HF and Spel-HF. The cysteine residue was deleted and the TAG codon was introduced at desired position (Table 3) by site directed mutagenesis using specific primers. All cloning and mutagenesis results were confirmed by Sanger sequencing (StarSEQ GmbH).

Table 3. Construction of NB-LUMABS variants

| Sensor name | SBiT1-LBiT interaction | SBiT2-LBiT interaction | Cys/TAG position |
|---|---|---|---|
| NB-LUMABS-1 | $K_d$=190μM | $K_d$ =2.5μM | Cys before SBiT2 |
| NB-LUMABS-2 | $K_d$ =190μM | $K_d$ =2.5μM | Cys after SBiT2 |
| NB-LUMABS-3 | $K_d$ =190μM | $K_d$ =2.5μM | Cys before and after SBiT2 |
| NB-LUMABS-4 | $K_d$ =190μM | $K_d$ =0.18μM | Cys before SBiT2 |
| NB-LUMABS-5 | $K_d$ =190μM | $K_d$ =190μM | Cys before SBiT2 |
| NB-LUMABS-6 | $K_d$ =2.5μM | $K_d$ =2.5μM | Cys before SBiT2 |
| NB-LUMABS-7 | $K_d$ =2.5μM | $K_d$ =0.18μM | Cys before SBiT2 |
| TRAS-NB- LUMABS-1 | $K_d$ =190μM | $K_d$ =2.5μM | Cys before SBiT2 |
| TRAS-NB- LUMABS-2 | $K_d$ =2.5μM | $K_d$ =0.18μM | Cys before SBiT2 |
| TRAS-NB- LUMABS-3 | $K_d$ =190μM | $K_d$ =190μM | Cys before SBiT2 |
| CTX-NB-LUMABS-1 | $K_d$=190 μM | $K_d$=2.5 μM | TAG before SBiT2 |

(continued)

| Sensor name | SBiT1-LBiT interaction | SBiT2-LBiT interaction | Cys/TAG position |
|---|---|---|---|
| CTX-NB-LUMABS-2 | $K_d$=190 $\mu$M | $K_d$=2.5 $\mu$M | TAG after SBiT2 |
| CTX-NB-LUMABS-3 | $K_d$=190 $\mu$M | $K_d$=2.5 $\mu$M | TAG before SBiT2 |

Protein expression and purification

Proteins were expressed and purified using standard protocols. Briefly, the appropriate pET28a plasmid was transformed into *E. coli* BL21 (DE3). The cells were cultured in LB medium containing 30 $\mu$g/ml kanamycin at 37 °C. Protein expression was induced at an $OD_{600}$ of 0.6 using 0.1 mM IPTG at 20 °C overnight. Cells were harvested and lysed by using the Bugbuster reagent (Novagen). Sensor proteins were purified using Ni-NTA affinity chromatography followed by Strep-Tactin purification according to the manufacturer's instructions. The protein concentration was determined by measuring the absorbance at 280 nm using extinction coefficient (calculated from the protein sequence). Protein purity was confirmed by SDS-PAGE. Purified proteins were stored at -80 °C until use.

The pET28a plasmids encoding CTX-NB-LUMABS was co-transformed into *E. coli* BL21 (DE3) together with a pEVOL vector encoding a tRNA/tRNA synthetase pair in order to incorporate para-azidophenylalanine (pAzF). The pEVOL vector was a gift from Peter Schultz (Addgene plasmid # 31186). Cells were cultured in 2YT medium (16 g peptone, 5 g NaCl, 10 g yeast extract per liter) containing 30 $\mu$g/mL kanamycin and 25 $\mu$g/mL chloramphenicol. Protein expression was induced using 0.1 mM IPTG and 0.2% arabinose in the presence of 1 mM pAzF (Bachem, F-3075.0001). The sensor proteins were purified as described above.

The pET28a plasmids encoding the initial pG-NB-LUMABS was ordered from GenScript, without the epitope sequence. Additionally, two pUC57 vectors were additionally ordered from GenScript encoding the two epitope sequences (SEQ ID NO: 23 and SEQ ID NO: 24) in the semiflexible linker. These epitope sequences were cloned into the pET28a+ vector using restriction ligation cloning using Kpnl and Spel restriction enzymes, and ligated using T4 ligase. The vectors were amplified by transforming them in *E. coli* NovaBlue bacteria (Novagen) and subsequent culturing. The vectors were extracted using a QIAprep spin miniprep kit (Qiagen). The correct incorporation of the semiflexible linkers with epitopes was confirmed by Sanger sequencing (StarSeq, Germany). The newly obtained pET28a+ vectors, including the epitopes, were transformed into *E. coli* BL21 (DE3) bacteria from Novagen, together with a pEVOL vector encoding the tRNA/tRNA synthetase for the incorporation of para-benzoylphenylalanine (pBPA) into the protein G. This pEVOL-pBpF vector was a gift from Peter Schultz (Addgene plasmid # 31190). The bacteria were cultured in LB medium, after the addition of 30 $\mu$g/mL kanamycin and chloramphenicol. The expression of the proteins was induced at an OD of 0.6 by 0.1 M IPTG (isopropyl-$\beta$-D-thiogalactopyranoside) and 0.2% arabinose, the unnatural amino acid pBPA (Bachem, F-2800.0001) was added simultaneously (1 mM). After overnight expression at 18 °C, the cultures were centrifuged at 10 000 xg for 10 minutes. The cells were lysed for 20 minutes at room temperature using BugBuster (20 mL/L) and Benzonase (20 $\mu$L/L). The lysed bacteria were centrifuged at 16 000 xg for 45 minutes at 4 °C. Purification of the supernatant was done using nickel-affinity and Strep-Tactin column chromatography. The purity of the sensor proteins was confirmed using SDS-PAGE gels and Q-ToF-MS.

Fluorophore labeling of protein

Purified NB-LUMABS protein was reduced with 1 mM tris-carboxyethylphosphine (TCEP) in 50 mM Tris-HCl (pH 7.4) at room temperature for 20 min. Sulfo-cyanine3 maleimide (Lumiprobe) dissolved in water (10 mg/ml) was then added in a 30-fold molar excess into the protein solution and incubated for 1 hour at room temperature followed by overnight incubation at 4 °C. Non-reacted dye was removed by using a PD10 desalting column and the conjugate was concentrated by using Amicon centrifuge filter of 10 kDa. The dye-to-protein ratio was determined by measuring absorbance at 280 nm and 553 nm and assuming extinction coefficients of 39,420 $M^{-1}$ $cm^{-1}$ and 162,000 $M^{-1}$ $cm^{-1}$ for the protein and dye, respectively. The Cy3-labeled NB-LUMABS protein was confirmed by Q-ToF LC-MS.

DBCO-Sulfo-Cy3 (Jena Bioscience, CLK-A140-1) was conjugated in a 40-fold molar excess to CTX-NB-LUMABS at room temperature overnight. Excess dye was removed by using Amicon centrifuge filter of 10 kDa. The dye-to-protein ratio was determined by measuring absorbance at 280 nm and 563 nm and using extinction coefficient (calculated from the protein sequence). The reaction product was analyzed by Q-TOF-MS.

Pure pG-NB-LUMABS (50 $\mu$M, 800 $\mu$L) in 50 mM TRIS pH 7 was bubbled with argon. 10 $\mu$L 100 mM TCEP (tris-carboxyethylphospine), with a final concentration of 1 mM was added and flushed with argon at room temperature for 20 minutes. The sulfo-Cy3-maleimide from Lumiprobe (1 mg) was dissolved in 100 $\mu$L MilliQ water. The dye was added to the protein solution (~15x fold excess of dye) and flushed with argon. This was placed at room temperature for 1 hour and overnight at 4 °C. The unreacted dye was removed using a PD-10 desalting column (GE Healthcare). The labeled protein was concentrated with a 3 kDa Amicon Ultra 0.5 mL centrifugal filter. The dye-to-protein ratio was measured using the NanoDrop (UV-VIS), absorption at 280 nm and 548 nm.

Photoconjugation of pG-NB-LUMABS

Photoconjugation reactions were performed using a Promed UVL-30 UV light source ($4{\times}9$ watt). 2 $\mu$M of antibody (HyTest) and 8 $\mu$M of pG-NB-LUMABS in PBS buffer (pH 7.4) was used to test the photoconjugation efficiency. The mixture was placed under UV light (365 nm), on ice, for 30, 60, or 90 minutes. The samples were then run on a SDS-PAGE gel to determine the photoconjugation efficiency.

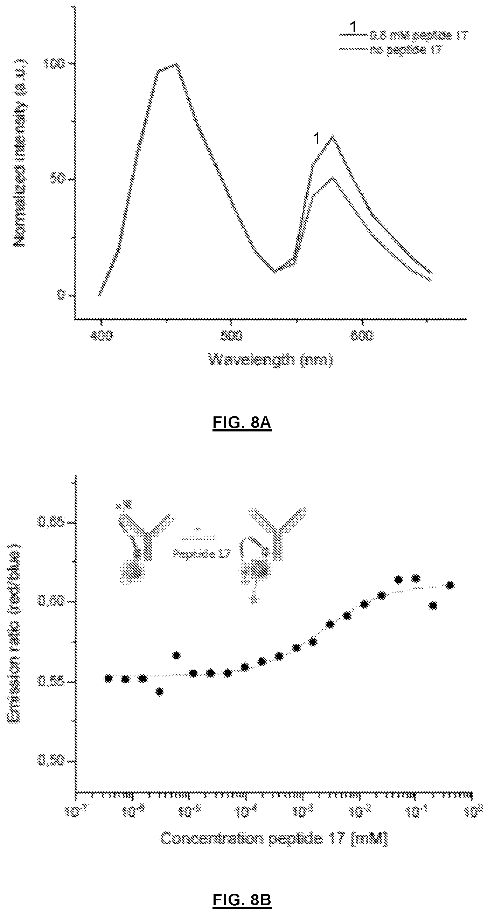

The excess of non-conjugated sensor protein was successfully removed using Size Exclusion Chromatography (SEC). Bioluminescence spectra show a substantial decrease in the Cy3 emission following conjugation of the pG-NB-LUMABS protein to its antibody, which is consistent with the anticipated binding of the peptide epitope to the antigen binding site, disrupting the LBit-Sbit2 interaction. Addition of the epitope peptide did result in an increase in BRET ratio, proving the feasibility of the sensor concept by showing the ability of the sensor to reversibly switch between the low- and high-BRET states as a result of competitive binding (Figure 8B).

Sensor characterization

Anti-HIV1-p17 antibody was obtained from Zeptometrix (clone 32/1.24.89). Trastuzumab (Herceptin, Roche) and cetuximab (Erbitux, Merck) were obtained via the Catherina hospital pharmacy in Eindhoven, the Netherlands. Antibody titrations to NB-LUMABS were performed in 100 $\mu$L PBS (pH 7.4, 1 mg/ml BSA) in PerkinElmer flat white 96-well Optiplate using a sensor concentration of 1 pM. Sensor of 100 pM was used for trastuzumab and cetuximab titration. After incubation of sensor and antibody for 2 hours, NanoGlo substrate was added at a final dilution of 1000-fold.

Anti-CRP169 binding peptide was titrated to antibody-conjugated pG-NB-LUMABS using a sensor concentration of 2 nM. After incubation of sensor and peptide for 45 minutes, NanoGlo substrate was added at a final dilution of 500-fold.

Luminescence spectra were recorded between 398 nm and 653 nm on Tecan Spark 10M plate reader with a step size of 15 nm, a bandwidth of 25 nm and an integration time of 1000 ms. Titrations were fit to eq 1 to obtain apparent $K_d$ value.

$$ER = \frac{P1[analyte]}{Kd,app+[analyte]} + P2 \qquad (Eq.\ 1)$$

P1 is the maximal change in emission ratio (ER) and P2 is the emission ration in absence of analyte. Dynamic range (DR) was calculated as:

$$DR = \frac{|P1|}{ERmin} * 100\% \qquad (Eq.\ 2)$$

Signal recording using digital camera

Into a white 96-well plate, 200 $\mu$L PBS buffer (pH 7.4, 1 mg/ml BSA) containing 100 pM CTX-NB-LUMABS, different concentrations of cetuximab and 1 $\mu$L NanoGlo substrate was added. The plate was placed into a Styrofoam box in a dark room to exclude the surrounding light. For blood plasma measurements, samples were prepared in 200 $\mu$L undiluted blood plasma with 5 nM sensor. The pictures were taken through a hole in the box using a SONY DSC-RX100 digital camera with exposure times of 10-30 s, F value of 1.8 and ISO value of 1600-6400. The images were analyzed by using ImageJ to calculate the ratio values between the average intensity in the blue and red color channel.

**Claims**

1.  Biosensor including a linker comprising a first end and a second end, wherein:

    - the first end of the linker is fused to one terminus of a first luciferase domain via a first binding domain; and
    - the second end of the linker is fused to a second luciferase domain via a second binding domain,

    wherein the biosensor optionally comprises a third luciferase domain fused to the other terminus of the first luciferase domain via a spacer,
    wherein the binding domains are configured to bind to a biomolecule,
    wherein the biosensor exists in two conformations, in which either the second or third luciferase domain binds to the first luciferase domain to form a complemented luciferase, and wherein a fluorophore is conjugated next to one of the second or third luciferase domains resulting in efficient Bioluminescence Resonance Energy Transfer (BRET) between the luciferase and the fluorophore in only one of the two conformations.

2.  Biosensor according to claim 1, wherein the third luciferase domain is fused to the other terminus of the first luciferase domain via a spacer.

3.  Biosensor according to claim 1 or 2, wherein the first luciferase domain is a large luciferase domain, preferably a large split luciferase fragment, and wherein the second and third luciferase domains are small luciferase domains, preferably small split luciferase fragments.

4.  Biosensor according to any of the preceding claims, wherein the linker is a semi-flexible linker.

5.  Biosensor according to any of the preceding claims, wherein the biomolecule comprises an antibody, antigen, protein or aptamer.

6.  Biosensor according to any of the preceding claims, wherein the first binding domain is configured to form a conjugated system with the biomolecule and wherein the second binding domain is configured to bind to a ligand binding site of the biomolecule.

7.  Biosensor according to claim 6, wherein the ligand binding site of the biomolecule comprises an antigen binding site of an antibody.

8.  Biosensor according to claim 6 or 7, wherein the biomolecule is covalently bound to the first binding domain of the biosensor.

9.  *In vitro* biomolecule-detecting method, comprising the steps of:

    - contacting a sample with the biosensor of any of claims 1-5;
    - determining the change of the biosensor's luminescence in the presence of a sample; and
    - determining the luminescence change in the presence of the sample to the quantitative and/or qualitative presence or absence of a biomolecule.

10. *In vitro* biomolecule-detecting method according to claim 9, wherein in the absence of a biomolecule the biosensor is in a biomolecule-free state that at least some of the first luciferase domain complements with the second luciferase domain.

11. *In vitro* biomolecule-detecting method according to claim 9 or 10, wherein binding between the binding domains of the biosensor and the biomolecule changes the equilibrium between the biomolecule-free state of the biosensor and a biomolecule-bound state of the biosensor such that the BRET between the luciferase and the fluorophore changed.

12. *In vitro* ligand-detecting method, comprising the steps of:

    - contacting the sample with the biosensor of any of claims 6-8;
    - determining the change of the biosensor's luminescence in the presence of a sample; and

- determining the luminescence change in the presence of the sample to the quantitative and/or qualitative presence or absence of a ligand.

**13.** *In vitro* ligand-detecting method according to claim 12, wherein in the absence of a ligand the biomolecule binds to the second binding domain disrupting the interaction of the second luciferase domain with the first luciferase domain.

**14.** *In vitro* ligand-detecting method according to claim 12 or 13, wherein in the presence of a ligand the biosensor is in a ligand-bound state allowing that at least some of the first luciferase domain complements with the second luciferase domain.

**15.** Kit of parts comprising the biosensor according to any of claims 1-8, wherein the biosensor comprises a first luciferase domain fused to a second luciferase domain via a semi-flexible linker containing two binding domains at the ends of the linker, and wherein the kit of parts further comprises a third luciferase domain.

**Patentansprüche**

**1.** Biosensor mit einem Linker (Verbinder), der ein erstes Ende und ein zweites Ende umfasst, wobei:

- das erste Ende des Linkers über eine erste Bindungsdomäne an einen Terminus (Endpunkt) einer ersten Luciferasedomäne fusioniert ist; und
- das zweite Ende des Linkers über eine zweite Bindungsdomäne mit einer zweiten Luciferasedomäne fusioniert ist,

wobei der Biosensor wahlweise eine dritte Luciferasedomäne umfasst, die über einen Spacer (Abstandhalter) mit dem anderen Terminus der ersten Luciferasedomäne fusioniert ist,
wobei die Bindungsdomänen dafür ausgelegt sind, an ein Biomolekül zu binden, wobei der Biosensor in zwei Konformationen vorliegt, in denen entweder die zweite oder die dritte Luciferasedomäne an die erste Luciferasedomäne bindet, um eine komplementäre Luciferase zu bilden, und wobei ein Fluorophor neben einer der zweiten oder dritten Luciferasedomänen konjugiert ist, was zu einem effizienten Biolumineszenz-Resonanzenergietransfer (BRET) zwischen der Luciferase und dem Fluorophor in nur einer der beiden Konformationen führt.

**2.** Biosensor gemäß Anspruch 1, wobei die dritte Luciferasedomäne über einen Spacer mit dem anderen Terminus der ersten Luciferasedomäne fusioniert ist.

**3.** Biosensor gemäß Anspruch 1 oder 2, wobei die erste Luciferasedomäne eine große Luciferasedomäne ist, vorzugsweise ein großes gespaltenes Luciferasefragment, und wobei die zweite und die dritte Luciferasedomäne kleine Luciferasedomänen sind, vorzugsweise kleine gespaltene Luciferasefragmente.

**4.** Biosensor gemäß einem der vorstehenden Ansprüche, wobei der Linker ein semiflexibler Linker ist.

**5.** Biosensor gemäß einem der vorstehenden Ansprüche, wobei das Biomolekül einen Antikörper, ein Antigen, ein Protein oder ein Aptamer umfasst.

**6.** Biosensor gemäß einem der vorstehenden Ansprüche, wobei die erste Bindungsdomäne dafür ausgelegt ist, ein konjugiertes System mit dem Biomolekül zu bilden, und wobei die zweite Bindungsdomäne dafür ausgelegt ist, an eine Ligandenbindungsstelle des Biomoleküls zu binden.

**7.** Biosensor gemäß Anspruch 6, wobei die Ligandenbindungsstelle des Biomoleküls eine Antigenbindungsstelle eines Antikörpers umfasst.

**8.** Biosensor gemäß Anspruch 6 oder 7, wobei das Biomolekül kovalent an die erste Bindungsdomäne des Biosensors gebunden ist.

**9.** *In-vitro*-Biomolekül-Detektionsverfahren, das die folgenden Schritte umfasst:

- Inkontaktbringen einer Probe mit dem Biosensor nach einem der Ansprüche 1-5;
- Bestimmen der Änderung der Lumineszenz des Biosensors in Gegenwart einer Probe; und

- Bestimmen der Änderung der Lumineszenz in Gegenwart der Probe zur quantitativen und/oder qualitativen Gegenwart oder Abwesenheit eines Biomoleküls.

10. *In-vitro*-Biomolekül-Detektionsverfahren gemäß Anspruch 9, wobei sich der Biosensor in Abwesenheit eines Biomoleküls in einem biomolekülfreien Zustand befindet, in dem wenigstens ein Teil der ersten Luciferasedomäne mit der zweiten Luciferasedomäne komplementär ist.

11. *In-vitro*-Biomolekül-Detektionsverfahren gemäß Anspruch 9 oder 10, wobei die Bindung zwischen den Bindungsdomänen des Biosensors und dem Biomolekül das Gleichgewicht zwischen dem biomolekülfreien Zustand des Biosensors und einem biomolekülgebundenen Zustand des Biosensors verändert, so dass sich der BRET zwischen der Luciferase und dem Fluorophor verändert.

12. *In-vitro*-Liganden-Detektionsverfahren, das die folgenden Schritte umfasst:

    - Inkontaktbringen der Probe mit dem Biosensor nach einem der Ansprüche 6-8;
    - Bestimmen der Änderung der Lumineszenz des Biosensors in Gegenwart einer Probe; und
    - Bestimmen der Änderung der Lumineszenz in Gegenwart der Probe zur quantitativen und/oder qualitativen Gegenwart oder Abwesenheit eines Liganden.

13. *In-vitro*-Liganden-Detektionsverfahren gemäß Anspruch 12, wobei in Abwesenheit eines Liganden das Biomolekül an die zweite Bindungsdomäne bindet und die Wechselwirkung der zweiten Luciferasedomäne mit der ersten Luciferasedomäne unterbricht.

14. *In-vitro*-Liganden-Detektionsverfahren gemäß Anspruch 12 oder 13, wobei sich der Biosensor in Anwesenheit eines Liganden in einem ligandengebundenen Zustand befindet, der ermöglicht, dass wenigstens ein Teil der ersten Luciferasedomäne mit der zweiten Luciferasedomäne komplementär ist.

15. Teilesatz, den Biosensor gemäß einem der Ansprüche 1-8 umfassend, wobei der Biosensor eine erste Luciferasedomäne umfasst, die mit einer zweiten Luciferasedomäne über einen semiflexiblen Linker fusioniert ist, der zwei Bindungsdomänen an den Enden des Linkers enthält, und wobei der Teilesatz ferner eine dritte Luciferasedomäne umfasst.

**Revendications**

1. Biocapteur comprenant un lieur comprenant une première extrémité et une deuxième extrémité, dans lequel :

    - la première extrémité du lieur est fusionnée à une extrémité terminale d'un premier domaine de luciférase via un premier domaine de liaison ; et
    - la deuxième extrémité du lieur est fusionnée à un deuxième domaine de luciférase via un deuxième domaine de liaison,

        dans lequel le biocapteur comprend éventuellement un troisième domaine de luciférase fusionné à l'autre extrémité terminale du premier domaine de luciférase via un espaceur,
        dans lequel les domaines de liaison sont configurés pour se lier à une biomolécule,
        dans lequel le biocapteur existe en deux conformations, dans lesquelles soit le deuxième soit le troisième domaine de luciférase se lie au premier domaine de luciférase pour former une luciférase complémentée, et où un fluorophore est conjugué à côté de l'un du deuxième ou du troisième domaine de luciférase conduisant à un Transfert d'Énergie par Résonance de Bioluminescence (BRET) efficace entre la luciférase et le fluorophore dans une seule des deux conformations.

2. Biocapteur selon la revendication 1, dans lequel le troisième domaine de luciférase est fusionné à l'autre extrémité terminale du premier domaine de luciférase via un espaceur.

3. Biocapteur selon la revendication 1 ou 2, dans lequel le premier domaine de luciférase est un grand domaine de luciférase, de préférence un grand fragment de luciférase divisée, et où les deuxième et troisième domaines de luciférase sont des petits domaines de luciférase, de préférence des petits fragments de luciférase divisée.

**4.** Biocapteur selon l'une des revendications précédentes, dans lequel le lieur est un lieur semi-flexible.

**5.** Biocapteur selon l'une des revendications précédentes, dans lequel la biomolécule comprend un anticorps, un antigène, une protéine ou un aptamère.

**6.** Biocapteur selon l'une des revendications précédentes, dans lequel le premier domaine de liaison est configuré pour former un système conjugué avec la biomolécule et dans lequel le deuxième domaine de liaison est configuré pour se lier à un site de liaison de ligand de la biomolécule.

**7.** Biocapteur selon la revendication 6, dans lequel le site de liaison de ligand de la biomolécule comprend un site de liaison d'antigène d'un anticorps.

**8.** Biocapteur selon la revendication 6 ou 7, dans lequel la biomolécule est liée de manière covalente au premier domaine de liaison du biocapteur.

**9.** Procédé de détection de biomolécule *in vitro,* comprenant les étapes de :

- mise en contact d'un échantillon avec le biocapteur de l'une des revendications 1 à 5 ;
- détermination du changement de la luminescence du biocapteur en présence d'un échantillon ; et
- détermination du changement de la luminescence en présence de l'échantillon afin de définir l'absence ou la présence quantitative et/ou qualitative d'une biomolécule.

**10.** Procédé de détection de biomolécule *in vitro* selon la revendication 9, dans lequel en l'absence d'une biomolécule, le biocapteur se trouve dans un état sans biomolécule dans lequel au moins une partie du premier domaine de luciférase se complémente avec le deuxième domaine de luciférase.

**11.** Procédé de détection de biomolécule *in vitro* selon la revendication 9 ou 10, dans lequel la liaison entre les domaines de liaison du biocapteur et la biomolécule change l'équilibre entre l'état sans biomolécule du biocapteur et un état lié à la biomolécule du biocapteur de sorte que la BRET entre la luciférase et le fluorophore change.

**12.** Procédé de détection de ligand *in vitro,* comprenant les étapes de :

- mise en contact de l'échantillon avec le biocapteur de l'une des revendications 6 à 8 ;
- détermination du changement de la luminescence du biocapteur en présence d'un échantillon ; et
- détermination du changement de la luminescence en présence de l'échantillon afin de définir l'absence ou la présence quantitative et/ou qualitative d'un ligand.

**13.** Procédé de détection de ligand *in vitro* selon la revendication 12, dans lequel en l'absence d'un ligand, la biomolécule se lie au deuxième domaine de liaison perturbant l'interaction du deuxième domaine de luciférase avec le premier domaine de luciférase.

**14.** Procédé de détection de ligand *in vitro* selon la revendication 12 ou 13, dans lequel en présence d'un ligand, le biocapteur se trouve dans un état lié au ligand permettant qu'au moins une partie du premier domaine de luciférase se complémente avec le deuxième domaine de luciférase.

**15.** Kit de composants comprenant le biocapteur selon l'une des revendications 1 à 8, dans lequel le biocapteur comprend un premier domaine de luciférase fusionné à un deuxième domaine de luciférase via un lieur semi-flexible contenant deux domaines de liaison au niveau des extrémités du lieur, et dans lequel le kit de composants comprend en outre un troisième domaine de luciférase.

FIG. 1A

FIG. 1B

FIG. 2A

FIG. 2B

**FIG. 3A**

**FIG. 3B**

FIG. 4

**FIG. 5A**

**FIG. 5B**

**FIG. 5C**

FIG. 6

R = methotrexate analog:

MTX1  MTX2  methotrexate

**FIG. 7**

FIG. 8A

FIG. 8B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62635053 B

### Non-patent literature cited in the description

- **BRENNAN et al.** *Protein Eng.,* 1994, vol. 7, 509
- **BENITO et al.** *J. Biol. Chem.,* 1996, vol. 271, 21251
- **FERRER-MIRALLES et al.** *Journal of Biological Chemistry,* 2001, vol. 276, 40087
- **GEDDIE et al.** *J. Mol. Biol.,* 2007, vol. 369, 1052
- **DE LAS HERAS et al.** *Biochem. Biophys. Res. Commun.,* 2008, vol. 370, 164
- **FRY et al.** *Biochem. Biophys. Res. Commun.,* 2008, vol. 372, 542
- **GOLYNSKIY et al.** *ChemBioChem,* 2010, vol. 11, 2264
- **BANALA et al.** *ACS Chem. Biol,* 2013, vol. 8, 2127
- **ARTS et al.** *Anal. Chem.,* 2016, vol. 88, 4525
- **VAN ROSMALEN et al.** *Anal. Chem.,* 2018
- **ARTS et al.** *ACS Sens.,* 2017, vol. 2, 1730
- **HALL, M.P. et al.** *ACS Chem Biol,* 2012, vol. 7, 1848
- **ARTS et al.** *Anal. Chem.,* 2016, vol. 88, 452
- **HALL et al.** *ACS Chem. Biol,* 2012, vol. 7, 1848
- **DIXON et al.** *ACS Chem. Biol,* 2016, vol. 11, 400
- **CHRISTOPHER et al.** *Anal. Biochem.,* 2017, vol. 522, 10
- **SASAKI et al.** *Virus Res.,* 2018, vol. 243, 69
- **DIXON et al.** *Sci Rep.,* 2017, vol. 7, 8186
- **VAN ROSMALEN.** *Anal. Chem.,* 2018, vol. 90, 3592
- **KRULJEC et al.** *Bioconjugate Chem,* 2017, vol. 28, 2009
- **DIXON et al.** *ACS Chem. Biol.,* 2016, vol. 11, 400
- **BANALA et al.** *ACS Chem. Biol.,* 2013, vol. 8, 212
- **ARTS et al.** *ACS Sens,* 2017, vol. 2, 1730
- **BRUNO et al.** *Cancer Chemother. Pharmacol.,* 2005, vol. 56, 361
- **VAN ROSMALEN et al.** *Anal. Chem.,* 2018, vol. 90, 3592